# EUROPÄISCHE PATENTSCHRIFT

(11) **EP 2 938 328 B1**
(45) Veröffentlichungstag und Bekanntmachung des Hinweises auf die Patenterteilung: **05.07.2023**
(21) Anmeldenummer: 13815516.3
(22) Anmeldetag: 23.12.2013
(51) Int. Cl.: A61K 9/20, A61K 9/24, A61K 47/38, A61K 31/135, A61K 31/138, A61K 31/495, A61K 31/522, A61P 25/00

(54) **MONOLITHISCHE ARZNEIFORM ZUR MODIFIZIERTEN FREISETZUNG EINER WIRKSTOFFKOMBINATION**
MONOLITHIC PHARMACEUTICAL FORM FOR MODIFIED RELEASE OF AN ACTIVE INGREDIENT COMBINATION
FORME PHARMACEUTIQUE MONOLITHIQUE POUR LIBERATION MODIFIE D'UNE COMBINAISON DE PRINCIPES ACTIFS

(30) Priorität: 27.12.2012 DE 102012113098; 01.02.2013 DE 102013101049
(43) Veröffentlichungstag der Anmeldung: 04.11.2015
(73) Patentinhaber: Hennig Arzneimittel GmbH&Co. Kg, 65439 Flörsheim am Main (DE)
(72) Erfinder: GERNOT, Francas, 67551 Worms (DE); PRZYKLENK, Karl-Heinz, 64521 Groß-Gerau (DE)
(74) Vertreter: Fuchs Patentanwälte Partnerschaft mbB
(86) Internationale Anmeldenummer: PCT/EP2013/077939
(87) Internationale Veröffentlichungsnummer: WO 2014/102253

(56) Entgegenhaltungen:
- WO-A1-2012/150246
- WO-A2-2006/099864
- WO-A2-2006/099865
- DE-A1-102011 075 354
- "SCHWINDELTHERAPIE MIT CINNARIZIN UND DIMENHYDRINAT KOMBINATIONSBEHANDLUNG IST EFFEKTIVER//THERAPY OF VERTIGO WITH CINNARIZINE AND DIMENHYDRINATE: COMBINATION TREATMENT IS MORE EFFECTIVE", TW NEUROLOGIE, PSYCHIATRIE, BRAUN, KARLSRUHE, DE, Bd. 11, Nr. 12, 1. Januar 1997 (1997-01-01), Seite 927/928, XP008036746, ISSN: 0935-3224
- Informationsblatt zu Gelucire® 50/13

## Beschreibung

Diese Erfindung betrifft monolithische Arzneiformen zur Freisetzung einer Wirkstoffkombination, die zur modifizierten Freisetzung von Wirkstoffen mit deutlich voneinander abweichenden Löslichkeitseigenschaften geeignet sind. Die Arzneiform umfasst hierzu wenigstens einen ersten Wirkstoff mit geringer Löslichkeit und einen zweiten Wirkstoff mit höherer Löslichkeit. insbesondere solche Wirkstoffe mit geringer Löslichkeit, deren Löslichkeit mit steigenden pH-Werten sinkt, profitieren von den erfindungsgemäßen Arzneiformen.

Die erfindungsgemäßen Arzneiformen ermöglichen eine optimal modifizierte Freisetzung der Wirkstoffkombination, so dass eine optimale therapeutische Wirkung und Wirkdauer sogar bei nur 2-mal oder 1-mal täglicher Einnahme einer erfindungsgemä-βen Arzneiform sichergestellt wird, wobei zugleich die Gefahr eines Dose-Dumpings insbesondere des zweiten Wirkstoffs deutlich vermindert ist. Die Arzneiformen ermöglichen eine optimale Freisetzung der Wirkstoffe unter Vermeidung zu hoher Plasmaspiegel genauso wie zu niedriger Plasmaspiegel unabhängig davon, ob die Arzneiform im nüchternen oder im nicht-nüchternen Zustand, erfindungsgemäß als "gesättigter Zustand" bezeichnet, eingenommen wird. Die Arzneiformen sind zudem einfach und kostengünstig herstellbar bei sehr hoher Lagerstabilität, wobei die Anforderungen gängiger Arzneibücher an die Qualität von Arzneiformen mindestens erfüllt sowie vorzugsweise übertroffen werden.

Grundsätzlich muss sich ein Wirkstoff, damit er resorbiert und in die Blutbahn gelangen sowie die gewünschte therapeutische Wirkung hervorrufen kann, zunächst im physiologischen Milieu des Magen-Darm-Traktes lösen. Löst er sich nicht, wird er nicht aus seiner Arzneiform frei gesetzt und die therapeutische Wirkung kann auch nicht eintreten.

Viele pharmazeutische Wirkstoffe sind gering löslich im physiologischen Milieu des Magen-Darm-Traktes. Zur Einteilung der Wirkstoffe nach deren Löslichkeit und Permeabilität wird in der Regel das biopharmazeutische Klassifizierungssystem (Biopharmaceutics Classification System, BCS) herangezogen, wonach es vier Klassen an Wirkstoffen gibt. Die Löslichkeit wird gemäß dieser Einteilung als hoch bewertet, wenn das Verhältnis der höchsten oralen Einzeldosis an Wirkstoff (in mg) zu seiner Löslichkeit (in mg/ml) weniger als 250 ml beträgt über den physiologischen pH-Bereich von pH 1 bis 7,5 bei 37°C. Der Wirkstoff muss sich also in seiner höchsten therapeutischen Einzeldosis in 250 ml Pufferflüssigkeit vollständig auflösen. Ein hohes Permeationsvermögen liegt vor, wenn der Wirkstoff zu mehr als 90 % im Dünndarm resorbiert wird. Hinsichtlich des Verfahrens zur Ermittlung der Löslichkeits- und Permeationseigenschaften wird auf die Leitlinien der amerikanischen Arzneimittelzulassungsbehörde (FDA) verwiesen (WHO 2005: Proposal to waive in vivo bioequivalence requirements for the WHO model list of essential medicines immediate release, solid oral dosage forms; Working document QAS/04.109/Rev.1). Wirkstoffe, die die Kriterien für eine hohe Löslichkeit nicht erfüllen, werden in die BCS-Klassen II und IV eingeteilt. Wirkstoffe der BCS-Klasse II weisen eine hohe Permeabilität auf im Gegensatz zu solchen der BCS-Klasse IV, die neben der geringen Löslichkeit auch eine geringe Permeabilität besitzen.

Die Resorption von Wirkstoffen der BCS-Klasse II wird also hauptsächlich durch die Löslichkeit und/oder die Lösungsgeschwindigkeit des Wirkstoffes über den physiologischen pH-Bereich kontrolliert. insbesondere bei solchen Wirkstoffen gilt es also, die Auflösung des Wirkstoffs unter den physiologischen Bedingungen zu steigern.

Besondere Schwierigkeiten bestehen zudem bei Wirkstoffen, die gering löslich sind und dabei zudem eine pH-abhängige Löslichkeit zeigen. Eine pH-abhängige Löslichkeit liegt vor, wenn die Löslichkeitseigenschaften des Wirkstoffs vom pH-Wert abhängen, sich also die Löslichkeit des Wirkstoffs mit dem pH-Wert ändert. insbesondere basische funktionelle Gruppen wie Aminogruppen, Guanidingruppen und stickstoffhaltige Heterozyklen im Wirkstoffmolekül können mit pH-abhängigen Löslichkeitseigenschaften einhergehen und die Formulierung des Wirkstoffs dadurch erschweren.

Schwierigkeiten bereitet insbesondere die Formulierung von Wirkstoffen, die schwache organische Säuren oder schwache organische Basen sind. Schwache organische Säuren weisen Säurefunktionen auf. Deshalb liegen sie im Darm deprotoniert und somit geladen vor. Deshalb lösen sie sich im basischen Milieu des Darms gut. Schwache organische Basen sind in diesem basischen Milieu nicht protoniert und somit ungeladen. Schwache organische Basen enthalten basische funktionelle Gruppen mit einem pK_{A}-Wert von höchstens 9, insbesondere einem pK_{A}-Wert von höchstens 8. Damit ist gemeint, dass die jeweils korrespondierende Säure der basischen funktionellen Gruppe den genannten pK_{A}-Wert aufweist. Für die schwachen organischen Basen gilt, dass sie sich typischerweise in saurem Milieu gut lösen. Die basische Gruppe ist in dem jeweiligen Molekül in saurem Milieu protoniert, sodass die schwache organische Base eine positive Ladung trägt. Ein solches saures Milieu herrscht typischerweise im nüchternen menschlichen Magen vor.

Schwache organische Basen sind also im pH-Bereich des Darms schlecht löslich. Der Darm, insbesondere der Dünndarm, ist aber der Ort, an dem typischerweise die Resorption von Wirkstoffen stattfindet und stattfinden soll, insbesondere aufgrund der großen Resorptionsfläche, die der Darm bietet.

Die oben skizzierten Umstände einer guten Löslichkeit im nüchternen Magen einerseits und einer schlechten Löslichkeit im Darm andererseits erschweren die Formulierung von solchen Wirkstoffen zusätzlich, insbesondere wenn eine modifizierte Freisetzung erwünscht ist. Es muss berücksichtigt werden, dass der pH-Wert im menschlichen Magen nicht immer sehr sauer ist. Nach der Einnahme einer Mahlzeit erreicht der pH-Wert durchaus neutrale Werte. Erst nach und nach sinkt der pH-Wert wieder. Das bedeutet für einen Wirkstoff mit schwach basischen Eigenschaften, dass er, wenn er nach einer Mahlzeit eingenommen wird, zunächst eine schlechte Löslichkeit im Magen aufweist, die sich mit der Zeit verbessert, weil der pH-Wert im Magen wieder saurer wird.

Es ist schwierig, eine modifizierte Freisetzung, insbesondere eine verlängerte Freisetzung, gering löslicher Wirkstoffe zu erzielen. Ein Ansatz, dem skizzierten Problem zu begegnen, ist die Formulierung von Arzneiformen mit verlängerter Freisetzung, indem die Arzneiform mit einem Überzug versehen wird, der den Wirkstoff nur langsam freigibt. Ebenso gibt es Ansätze, solche Wirkstoffe in einer Matrix zu dispergieren, die nur langsam erodiert.

Bei schwach basischen Wirkstoffen tritt das Problem hinzu, dass sich der Wirkstoff - wie bereits beschrieben - im alkalischen Milieu des Darmes nur schlecht löst. Für eine verlängerte, möglichst lineare Freisetzung ist es aber erforderlich, dass sich der Wirkstoff kontinuierlich löst. in der Praxis führt dies dazu, dass Arzneiformen, die einen schwach basischen Wirkstoff mit schlechter Darmlöslichkeit verlängert freisetzen sollen, diesem Anspruch zwar zunächst (im Magen) gerecht werden, sobald die Arzneiform aber den Darm erreicht, eine so schlechte Freisetzung erzielen, dass die Anwendung nicht zielführend ist.

Ein weiteres Problem tritt auf, wenn zwei Wirkstoffe mit deutlich voneinander abweichendem Lösungsverhalten aus einer Arzneiform in gleicher Weise, insbesondere modifiziert, freigesetzt werden sollen. Bei besser löslichen Wirkstoffen, insbesondere solchen mit permanenter Ladung, tritt das Problem auf, dass die Freisetzung dieser Wirkstoffe verzögert werden muss. insbesondere im gesättigten Zustand besteht die Gefahr, dass der besser lösliche Wirkstoff in zu hoher Menge zu schnell in den Magen freigesetzt wird (Dose Dumping), mit der Folge von toxischen Plasmaspiegeln sowie einer möglichen Reizung der Magenschleimhaut. Dies ist besonders gefährlich, wenn der besser lösliche Wirkstoff zudem eine hohe Eliminationshalbwertszeit aufweist, d.h. eine Eliminationshalbwertszeit von mehr als 2 Stunden. Es muss also hinsichtlich des Wirkstoffes mit geringer Löslichkeit eine Förderung der Auflösung und Freisetzung jedenfalls im Darm und hinsichtlich des besser löslichen Wirkstoffes eine Retardierung erzielt werden. Dabei muss sichergestellt werden, dass sich eine ausreichende Menge des geringer löslichen Wirkstoffs vorzugsweise im Magen löst. Die Auflösung dieses Wirkstoffs darf dabei allerdings auch nicht zu stark gefördert werden, denn auch dann kann es insbesondere bei längerer Verweilzeit im Magen zur Akkumulation hoher Wirkstoffmengen mit der Folge eines zu steilen Anstieges des Plasmaspiegels und einer Reizung der Magenschleimhaut kommen.

Eine Herausforderung besteht ferner darin, eine Kombination von Wirkstoffen mit deutlich voneinander abweichenden Löslichkeitseigenschaften so bereitzustellen, dass eine mehrmals tägliche Gabe optimal nachgeahmt wird und die Dosisintervalle somit reduziert werden können, idealerweise auf eine 2-mal oder 1-mal tägliche Einnahme der Arzneiform. Gerade bei Langzeittherapien kann dabei ein langsamer und andauernder Anstieg der Plasmaspiegel der Wirkstoffe aus der Arzneiform erwünscht sein, auch um eine Kumulation mit Plasmaspiegeln der vorherigen Dosis im Blut zu verhindern und Plasmaspiegelspitzen oder stark schwankende Plasmaspiegel zu vermeiden.

Zusätzliche Schwierigkeiten bestehen, wenn die Wirkstoffe nicht unbeschränkt lagerstabil sind und/oder neben den ungünstigen Löslichkeitseigenschaften sonstige Wirkstoffeigenschaften aufweisen, die die Verarbeitung zusätzlich erschweren. Eine Verbindung ist nicht unbeschränkt lagerstabil, wenn ihre Lagerung besondere Vorsichtsmaßnahmen erfordert beispielsweise das Erfordernis einer Lagerung vor Licht geschützt und/oder das Erfordernis einer Lagerung des Wirkstoffs im dicht verschlossenen Behältnis. Sonstige Wirkstoffeigenschaften, die die Verarbeitung zusätzlich erschweren und damit weitere besondere Anforderungen an Arzneiformulierungen stellen, sind beispielsweise ein schlechter Geschmack oder unangenehmer Geruch eines Wirkstoffs.

Bisherige Arzneiformen, in denen mehrere Wirkstoffe mit deutlich voneinander abweichenden Löslichkeitseigenschaften verarbeitet sind, erfordern regelmäßig aufwändige Herstellungsprozesse umfassend zahlreiche Prozessschritte und die Arzneiformen sind insofern nicht kostengünstig und schnell herstellbar. Zumeist werden die Wirkstoffe mit den abweichenden Löslichkeitseigenschaften getrennt verarbeitet und in abgetrennten Arzneiformen oder in multipartikulären Arzneiformen in verschiedenen Untereinheiten bereitgestellt, weil die Unterstützung der Freisetzung der Wirkstoffe jeweils unterschiedliche Zusammensetzungen erfordert. Nur vereinzelt werden die Wirkstoffe mit abweichenden Löslichkeitseigenschaften in einer einzigen Untereinheit bereitgestellt, jedoch sind in solchen multipartikulären Arzneiformen mehrere Untereinheiten mit abweichendem Freisetzungsverhalten erforderlich, um die Freisetzung der Wirkstoffe derart zu gestalten, dass Dosisintervalle verlängert werden können. Letzteres erfordert eine aufwändige und vielstufige Herstellung. Zudem sind solche Arzneiformen regelmäßig recht groß und damit nur schwer für den Patienten schluckbar. Dies kann einen nachteiligen Einfluss auf die Patientencompliance haben.

Die Dokumente DE102011075354 und WO2006/099864 offenbaren Arzneiformen zur Abgabe mehrerer Wirkstoffe.

Es besteht also ein Bedarf an Arzneiformen, die eine therapeutisch optimale Modifikation der Freisetzung wenigstens zweier Wirkstoffe mit deutlich voneinander abweichenden Löslichkeitseigenschaften gleichermaßen sicherstellen und dabei die Gefahr eines Dose Dumping der Wirkstoffe deutlich vermindern. Dabei soll es möglich sein, selbst schwach basische Wirkstoffe optimal freizusetzen. Mit "frei setzen" ist in diesem Zusammenhang gemeint, dass der Wirkstoff am Ort der Resorption, also im Dünndarm, verfügbar gemacht wird. Die Arzneiformen sollen einfach und kostengünstig herstellbar sein und eine optimale Lagerstabilität selbst im Falle nicht unbeschränkt lagerstabiler Wirkstoffe sowie eine geeignete Größe aufweisen. Es soll zudem möglich sein, selbst Wirkstoffe mit für die Verarbeitung ungünstigen sonstigen Wirkstoffeigenschaften optimal darzubieten. insbesondere sollen die Arzneiformen eine Reduktion der Anzahl täglicher Dosen an Wirkstoffen ermöglichen und damit auf eine verbesserte Compliance hinwirken.

Die oben skizzierten Aufgaben werden durch die Gegenstände der Patentansprüche gelöst. Erfindungsgemäß sind Arzneiformen, die wenigstens einen ersten Wirkstoff und einen zweiten Wirkstoff aufweisen, wobei der erste Wirkstoff vorzugsweise wenigstens eine basische funktionelle Gruppe aufweist.

Die Verweise auf therapeutische oder chirurgische Behandlungsverfahren oder In-vivo-Diagnostizierverfahren in dieser Beschreibung sind als Verweis auf Verbindungen, pharmazeutische Zubereitungen und Arzneimittel zur Anwendung in diesen Verfahren zu verstehen.

Der erste Wirkstoff weist bei einem pH-Wert von 7 und 22°C eine Löslichkeit von weniger als 0,01 mg/ml auf. Der zweite Wirkstoff weist bei pH 7 und 22°C eine Löslichkeit auf, die die Löslichkeit des ersten Wirkstoffs bei pH 7 und 22°C um wenigstens eine Zehnerpotenz überschreitet.

Außerdem umfassen die Arzneiformen erfindungsgemäß wenigstens einen Emulgator, wobei der Emulgator durch Reaktion von Mono-, Di-, und/oder Triglyceriden mit Polyethylenglykol hergestellt wird, wobei die Polyethylenglykolkette den hydrophilen Teil des Emulgators darstellt und mit einem lipophilen Teil verbunden ist, und die Polyethylenglykolkette wenigstens 4 Kettenglieder aufweist, wobei der Emulgator ein Strukturmotiv folgender Formel aufweist

R¹-O-[CH₂-CH₂-O]ₙ-R²

wobei R¹ und R² unabhängig voneinander Wasserstoff, Alkyl, Glycerid oder Polyalkylenoxid sind und n eine ganze Zahl von wenigstens 4 ist. Ebenso ist in den Arzneiformen erfindungsgemäß wenigstens ein Retardierungsmittel enthalten in einem solchen Anteil, dass das Masseverhältnis von zweitem Wirkstoff zu Retardierungsmittel weniger als 1:1 beträgt. Die Summe der Anteile an Retardierungsmittel und Emulgator an erfindungsgemäßen Arzneiformen beträgt wenigstens 40 Gew.-%.

Die Arzneiformen sind erfindungsgemäß perorale feste Arzneiformen. Das bedeutet, dass sie zur oralen Einnahme bestimmt sind. Perorale feste Arzneiformen können leicht vom Patienten selbst appliziert werden und es besteht eine recht große Patientenakzeptanz für solche Arzneiformen. Es kann sich bei den erfindungsgemäßen Arzneiformen beispielsweise um eine Kapsel oder eine Tablette handeln, bevorzugt eine Tablette.

Es handelt sich bei den erfindungsgemäßen Arzneiformen um monolithische Arzneiformen. Darunter werden erfindungsgemäß Arzneiformen verstanden, die durch Erosion und/oder Abbau während der Magen-Darm-Passage immer kleiner werden. Monolithische Arzneiformen sind von multipartikulären Arzneiformen abzugrenzen, die nach Einnahme oder im Magen in mehrere diskrete Untereinheiten mit einer Größe üblicherweise unter 2 mm zerfallen, wie Minitabletten oder Pellets, mit jeweils spezifischen Eigenschaften und Freisetzungsprofilen, die den Pylorussphinkter auch im geschlossenen Zustand passieren können. Dagegen verweilen monolithische Arzneiformen solange im Magen, bis durch Erosion und/oder Auflösung eine bestimmte Größe unterschritten ist und die Arzneiform durch den Pylorussphinkter passt und/oder der Magen sich mittels sogenannter Housekeeper-Kontraktionen entleert. Die Ausgestaltung als monolithische Arzneiform ermöglicht eine schnelle und einfache sowie kostengünstige Herstellung im Gegensatz zu Herstellungsverfahren für multipartikuläre Arzneiformen. Es ist dabei zu berücksichtigen, dass natürlich aufwändige Herstellungsverfahren stets auch die Wirkstoffe belasten und deren Zersetzung oder generell die Bildung von Verunreinigungen fördern können. Monolithische Arzneiformen sind also keine multipartikulären Arzneiformen. insbesondere wird die erfindungsgemäße Arzneiform nicht durch Verpressen gleich großer Untereinheiten mit jeweils spezifischen Eigenschaften und Freisetzungsprofilen, insbesondere Pellets, hergestellt. Die erfindungsgemäße monolithische Arzneiform zerfällt nach der Einnahme im Magen nicht in gleich große Untereinheiten, insbesondere Pellets.

in besonders bevorzugten Ausführungsformen handelt es sich bei den hier beschriebenen Arzneiformen um Magenverweilformen. Das bedeutet, dass die Arzneiformen sich im Magen nicht so stark auflösen und/oder erodieren, dass sie den Magen schnell durch den Pylorussphinkter verlassen können. Vorzugsweise ist eine Magenverweilform eine solche, die für wenigstens 2 Stunden, weiter bevorzugt wenigstens 3 Stunden im Magen des Patienten verweilt, bevor sie in den Darm gelangt. Dies wird erfindungsgemäß dadurch erzielt, dass die Arzneiformen so ausgestaltet sind, dass sie sich nur sehr langsam auflösen. Dadurch behalten sie über einen längeren Zeitraum eine Größe, die es ihnen nicht erlaubt, den Magen zu verlassen.

Da die Arzneiformen sich vorzugsweise nur sehr langsam auflösen, bleiben sie bevorzugt im Magen, bis sich dieser weitgehend entleert hat. Der Speisebrei ist dann zu einem großen Anteil über den Pylorussphinkter in den Dünndarm gelangt. Der Magen leert sich in einem solchen Moment mittels sogenannter Housekeeper-Kontraktionen. Dadurch gelangen auch Körper, die normalerweise nicht über den Pylorussphinkter in den Dünndarm übergehen, dorthin. Die Arzneiformen werden dann weiter im Dünndarm die Wirkstoffe freisetzen. So wird eine nahezu lineare Freisetzung des ersten und des zweiten Wirkstoffs möglich.

Die erfindungsgemäßen Arzneiformen setzen den ersten Wirkstoff und den zweiten Wirkstoff modifiziert frei. Unter "modifizierter Freisetzung" wird dabei erfindungsgemäß eine verzögerte, pulsierende, verlängerte Freisetzung der Wirkstoffe oder Mischungen dieser Freisetzungsarten verstanden. Eine verzögerte Freisetzung bedeutet eine zeitliche Verzögerung der Freisetzung des Wirkstoffs nach der Einnahme im Vergleich mit sofort freisetzenden Arzneiformen, also ein späterer Beginn der Freisetzung. Dies ist vorzugsweise gegeben, wenn nach 60 Minuten weniger als 30% an Wirkstoff in das jeweilige Prüfmedium freigesetzt sind, vorzugsweise weniger als 25%, mehr bevorzugt weniger als 23% und besonders bevorzugt weniger als 20%. Besonders bevorzugt sind nach 120 Minuten höchstens 40%, bevorzugt höchstens 35% und besonders bevorzugt höchstens 30% an Wirkstoff aus der Arzneiform freigesetzt. Die Messung kann nach üblichen Methoden der Freisetzungsmessung erfolgen, insbesondere der Methoden nach Europäischen Arzneibuch oder des Amerikanischen Arzneibuchs (USP), bevorzugt mit der Bio-Dis-Apparatur (200 ml Prüfmedium, 25 dpM, 37°C).

Der Fachmann ist in der Lage, das Prüfmedium in Abhängigkeit vom Wirkstoff und der konkreten Freisetzungskinetik zu wählen. in Frage kommen beispielsweise Prüfmedien gängiger Arzneibücher, insbesondere des Europäischen Arzneibuchs oder des Amerikanischen Arzneibuchs. Als besonders geeignet für die Ermittlung der Freisetzung der erfindungsgemäßen Arzneiform haben sich Prüfmedien ausgewählt aus 0,1 N (0,1 mol/l) Salzsäurelösung (Ph. Eur. 7), FeSSIF-Puffer nach Dressman et. al., FaSSIF-Puffer nach Dressman et. al., Pufferlösung pH 3,0 R (Ph. Eur. 7), FaSSGF-Puffer nach Dressman et. al. und FeSSGF-Puffer nach Dressman et. al. erwiesen. Bevorzugt werden wenigstens zwei der genannten Prüfmedien für die Ermittlung der Freisetzung der erfindungsgemäßen Arzneiform kombiniert. Unter der Kombination von Prüfmedien ist zu verstehen, dass diese zeitlich nacheinander eingesetzt werden. Ein Prüfmedium wird also nach einer bestimmten Zeit, beispielsweise nach 60 Minuten, durch ein anderes Prüfmedium ersetzt. Dies ermöglicht eine optimale Nachahmung physiologischer Vorgänge bei der Einnahme eines Arzneimittels. Damit sind die Ergebnisse weitgehend auf die in-vivo-Freisetzung des Wirkstoffs übertragbar.

Eine pulsierende Freisetzung umfasst die Freisetzung des Wirkstoffs mit mehr als einer Phase, wobei eine Phase ein Freisetzungsimpuls ist.

Eine verlängerte Freisetzung ist erfindungsgemäß eine Freisetzung des Wirkstoffs über wenigstens 5 Stunden, weiter bevorzugt wenigstens 6 Stunden, noch weiter bevorzugt wenigstens 8 Stunden und idealerweise sogar wenigstens 9 Stunden. Es wird also auch noch nach wenigstens 5, weiter bevorzugt wenigstens 6, noch weiter bevorzugt wenigstens 8 und idealerweise wenigstens 9 Stunden Wirkstoff aus der Arzneiform in das Prüfmedium freigesetzt. Damit kann zum einen ein therapeutischer Plasmaspiegel für ausreichende Zeit während der Nacht sichergestellt werden, so dass der Patient nicht in der Nacht gezwungen ist, eine Dosis einzunehmen. Ebenso können die Dosisintervalle am Tag deutlich verlängert werden, was sich positiv auf die Patientencompliance auswirkt.

Bevorzugt setzt die erfindungsgemäße Arzneiform die Wirkstoffe verlängert frei in Kombination mit einer verzögerten Freisetzung der Wirkstoffe. Die Freisetzung des ersten Wirkstoffs und des zweiten Wirkstoffs setzt also verzögert ein und ist zugleich verlängert. Es werden also über eine längere Zeit ausreichend hohe Plasmaspiegel aufrechterhalten. Eine mehrmals tägliche Gabe des ersten und/oder des zweiten Wirkstoffs kann daher durch Einnahme der erfindungsgemäßen Arzneiform auf eine 2-mal tägliche Einnahme, vorzugsweise eine 1-mal tägliche Einnahme reduziert werden.

Die modifizierte Freisetzung des ersten und des zweiten Wirkstoffs erfolgt vorzugsweise unabhängig voneinander, d.h. die Freisetzung des ersten und des zweiten Wirkstoffs wird im Wesentlichen durch verschiedene Freisetzungsmechanismen innerhalb der Arzneiform gesteuert. An der Freisetzung des ersten Wirkstoffs ist der Emulgator beteiligt, während an der Freisetzung des zweiten Wirkstoffs das Retardierungsmittel und der Emulgator beteiligt sind. Dies hat den Vorteil, dass die Gefahr eines Dose Dumping des zweiten Wirkstoffs insbesondere bei längerer Verweilzeit im Magen noch deutlicher vermindert und die Auslösung des ersten Wirkstoffs im Magen optimal unterstützt werden kann.

Die erfindungsgemäßen Arzneiformen haben eine verlängerte Verweildauer im Magen, so dass sie hinreichend lang im Magen sind, dass sich ein signifikanter Anteil des ersten Wirkstoffes lösen kann, gleichzeitig aber ein Dose-Dumping des ersten und zweiten Wirkstoffs verhindert wird.

Die erfindungsgemäßen Arzneiformen sind vorzugsweise so gestaltet, dass sie eine Masse von 1400 mg, weiter bevorzugt 1200 mg und besonders bevorzugt 1000 mg sowie ganz besonders bevorzugt 900 mg nicht übersteigen. Ihre Masse beträgt formulierungsbedingt bevorzugt wenigstens 400 mg, weiter bevorzugt wenigstens 450 mg und noch mehr bevorzugt wenigstens 500 mg.

Die Arzneiformen dieser Erfindung können in Form einer Tablette oder Kapsel ausgestaltet sein. Bevorzugte Formen sind Tabletten. Die Arzneiform hat vorzugsweise an wenigstens einer Stelle einen Durchmesser von mehr als 5 mm, insbesondere mehr als 7 mm und besonders bevorzugt wenigstens 8 mm. Dadurch wird sichergestellt, dass die Arzneiform den Magen nicht sofort durch den Pylorussphinkter verlassen kann, sondern noch eine Weile im Magen bleibt, bevorzugt sind dies wenigstens 2 Stunden, weiter bevorzugt wenigstens 3 Stunden.

Der Magen entleert sich nach der Nahrungsaufnahme nicht sofort, sondern gibt den Speisebrei (Chymus) nur nach und nach an den Dünndarm ab. Diese physiologische Gegebenheit kann sich die erfindungsgemäße Arzneiform zu Nutze machen. Der erste Wirkstoff ist vorzugsweise eine schwache Base, und weist somit bei einem niedrigen pH-Wert, also im sauren Milieu, eine bessere Löslichkeit auf. Im Magen herrscht typischerweise ein niedriger pH-Wert. Nach dem Essen steigt der pH-Wert allerdings auf höhere Werte an, z.B. um pH 5. Erst nach und nach sinkt der pH wieder auf niedrigere Werte. Das heißt, dass sich der erste Wirkstoff nach der Nahrungsaufnahme zunächst schlecht lösen würde, weil der pH-Wert zu hoch ist. Die erfindungsgemäße Arzneiform ermöglicht nun aber die Auflösung eines Teils des ersten Wirkstoffes durch die hierin beschriebenen Maßnahmen.

Durch die Magenbewegung mischt sich der freigesetzte erste Wirkstoff mit dem Chymus und wird dann nach und nach an den Dünndarm abgegeben. Der Wirkstoff wird dann im proximalen Dünndarm, also kurz nach dem Verlassen des Magens, resorbiert. Der Magen dient somit als zusätzliches Retardierungsmittel für den ersten Wirkstoff. Gleichzeitig löst sich auch der besser lösliche zweite Wirkstoff.

Sobald der pH-Wert im Magen einen niedrigeren Wert, z.B. etwa pH 3, erreicht hat, löst sich der erste Wirkstoff, der bevorzugt eine schwache Base ist, besser. Es besteht dann die Gefahr, dass zu viel erster Wirkstoff in Lösung geht. Daher wird die Freisetzung des ersten Wirkstoffs in der erfindungsgemäßen Arzneiform vorzugsweise verzögert und verlangsamt. An der Verzögerung und Verlangsamung der Freisetzung des ersten Wirkstoffs ist der Emulgator beteiligt, bevorzugt wird die Freisetzung des ersten Wirkstoffs sowohl durch die Magenbewegung also auch durch den Emulgator gesteuert.

Besonders bevorzugt erfolgt auch die Freisetzung des zweiten Wirkstoffs verzögert und verlängert. An der Modifikation der Freisetzung des zweiten Wirkstoffs sind der Emulgator und das Retardierungsmittel beteiligt, bevorzugt erfolgt die Steuerung der Freisetzung des zweiten Wirkstoffs durch den Emulgator und das Retardierungsmittel.

Der erste Wirkstoff weist in wässriger Lösung bei pH 7 und 22°C eine Löslichkeit von weniger als 0,01 mg/ml auf. Der erste Wirkstoff hat also eine geringe Löslichkeit, er ist in Übereinstimmung mit der Löslichkeitsklassifikation des Europäischen Arzneibuchs "praktisch unlöslich" (Ph.Eur. 7, 2011). Bevorzugt beträgt die Löslichkeit bei pH 7 und 22°C in wässriger Lösung höchstens 0,005 mg/ml, weiter bevorzugt höchstens 0,001 mg/ml und noch mehr bevorzugt sogar weniger als 0,001 mg/ml.

Unter Löslichkeit wird die Konzentration des gelösten Stoffes in einer gesättigten Lösung bei einer bestimmten Temperatur verstanden. Die Löslichkeit des Wirkstoffs bezieht sich erfindungsgemäß auf die Löslichkeit in wässriger Lösung bei 22°C unter Normaldruck. Diese Temperaturangabe sowie alle nachfolgenden Temperaturangaben umfassen erfindungsgemäß den konkret angegebenen Wert einschließlich einer Spanne von +/- 3°C um den angegebenen Temperaturwert. Nachfolgende Löslichkeitsangaben beziehen sich, soweit nichts anderes angegeben ist, auf die Löslichkeit unter Normaldruck. Die Bestimmung erfolgt in Standard- beziehungsweise Pufferlösungen des Europäischen Arzneibuchs (Ph. Eur. 7) mit entsprechendem pH-Wert.

Die wässrige Lösung zur Erzielung eines pH-Wertes von 7 umfasst einen Phosphatpuffer und Wasser. Die Zusammensetzung ist dem Europäischen Arzneibuch zu entnehmen (Ph. Eur. 7; Phosphat-Pufferlösung pH 7.0 R). Die Messung des pH-Wertes erfolgt mit dem Fachmann bekannten Verfahren, beispielsweise mittels eines kommerziell erhältlichen pH-Meters.

Zur Löslichkeitsbestimmung werden dem Fachmann bekannte Verfahren eingesetzt wie die Bodensatzmethode, die Temperaturmethode oder die Verteilungsmethode. insbesondere geeignet ist die Verteilungsmethode, bei der der Wirkstoff in einem zweiten Lösungsmittel gelöst wird, welches nicht mischbar ist mit der jeweiligen wässrigen Lösung, für die die Löslichkeit des ersten Wirkstoffs ermittelt werden soll. Die beiden Flüssigkeiten werden bis zur Gleichgewichtseinstellung intensiv geschüttelt und dann getrennt.

Der erste Wirkstoff ist vorzugsweise ausgewählt aus der BCS-Klasse II oder BCS-Klasse IV, besonders bevorzugt ist der erste Wirkstoff ein Wirkstoff der BCS-Klasse II. Gerade solche Wirkstoffe profitieren besonders von den erfindungsgemäßen Arzneiformen, weil die Auflösung des ersten Wirkstoffs besonders gefördert wird.

Der erste Wirkstoff weist vorzugsweise wenigstens eine basische funktionelle Gruppe auf. Gerade solche funktionellen Gruppen, die gewöhnlich mit pH-abhängiger Löslichkeit einhergehen, bereiten bei der Formulierung von Arzneiformen bislang erhebliche Schwierigkeiten.

Eine Gruppe ist insbesondere basisch, wenn sie im ungeladenen Zustand des Wirkstoffs Protonen aufnehmen kann. im Sinne der Erfindung werden nur solche Gruppen als basische funktionelle Gruppen bezeichnet, die einen pK_{A}-Wert von höchstens 9, weiter bevorzugt einen pK_{A}-Wert von höchstens 8,5 und mehr bevorzugt einen pK_{A}-Wert von höchstens 8 haben. Damit ist gemeint, dass die jeweils korrespondierende Säure der basischen funktionellen Gruppe einen solchen pK_{A}-Wert aufweist.

Die basische funktionelle Gruppe ist bevorzugt ausgewählt aus aliphatischen stickstoffhaltigen Gruppen oder einem stickstoffhaltigen Heterozyklus. Unter dem Begriff "stickstoffhaltiger Heterozyklus" werden im Sinne dieser Erfindung stickstoffhaltige Gruppen verstanden, die Bestandteil von aromatischen Kohlenwasserstoffen sind. Die aromatischen Kohlenwasserstoffe können neben der stickstoffhaltigen Gruppe noch weitere Heteroatome als Ringglieder umfassen. Bevorzugte "stickstoffhaltige Heterozyklen" sind aber solche, die neben Stickstoff nur Kohlenstoffatome als Ringglieder enthalten. Aliphatische stickstoffhaltige Gruppen sind vorzugsweise ausgewählt aus Aminogruppen und Guanidingruppen. Der Begriff "Aminogruppe" umfasst primäre, sekundäre und tertiäre Aminogruppen. Auch Stickstoffatome in gesättigten oder teilweise ungesättigten cyclischen Kohlenwasserstoffen, die neben dem Stickstoffatom noch weitere Heteroatome einschließlich weiterer Stickstoffatome als Ringglieder aufweisen können, stellen eine Aminogruppe dar.

Ganz besonders bevorzugt ist die basische funktionelle Gruppe ausgewählt aus Aminogruppe, Guanidingruppe und stickstoffhaltiger Heterozyklus. Noch mehr bevorzugt ist die basische funktionelle Gruppe eine Aminogruppe. Solche Wirkstoffe profitieren aufgrund der ausgeprägten pH-abhängigen Löslichkeit besonders von den erfindungsgemäßen Arzneiformen.

Es ist bevorzugt, dass wenigstens eine basische funktionelle Gruppe des ersten Wirkstoffs einen pK_{A}-Wert von wenigstens 4,5, weiter bevorzugt von wenigstens 5,8 und mehr bevorzugt von wenigstens 6,5 hat. Damit ist gemeint, dass die korrespondierende Säure zu der basischen funktionellen Gruppe einen solchen pK_{A}-Wert aufweist. Solche Wirkstoffe zeigen besonders ausgeprägte pH-abhängige Löslichkeitseigenschaften, die eine Formulierung bislang besonders erschwert haben.

Ganz besonders profitieren solche Wirkstoffe von der erfindungsgemäßen Arzneiform, die mehrwertige Basen sind, also wenigstens zwei basische funktionelle Gruppen aufweisen. Besonders bevorzugt enthält der erste Wirkstoff daher wenigstens zwei basische funktionelle Gruppen, noch mehr bevorzugt genau zwei basische funktionelle Gruppen. Noch weiter bevorzugt weist der erste Wirkstoff zwei Aminogruppen auf.

Vorzugsweise handelt es sich also bei dem ersten Wirkstoff um einen schwach basischen Wirkstoff, also einen solchen, der wenigstens eine basische funktionelle Gruppe mit einem pK_{A}-Wert von höchstens 9, weiter bevorzugt von höchstens 8,5 und mehr bevorzugt von höchstens 8 aufweist. Wie ausgeführt bezieht sich die Angabe des pK_{A}-Wertes auf die korrespondierende Säure der basischen funktionellen Gruppe.

Dadurch ist der erste Wirkstoff, wenn er mit Magensaft in Kontakt kommt, üblicherweise protoniert und somit in diesem sauren Milieu wasserlöslich. Bei höheren pH-Werten im Darm von 6 und mehr hingegen kann sich der Wirkstoff nur schlecht auflösen. Das trifft besonders auf Wirkstoffe zu, die keine sauren Gruppen aufweisen.

Weiter bevorzugt enthält der erste Wirkstoff also keine sauren Gruppen. Saure Gruppen sind solche, die im ungeladenen Zustand des Wirkstoffs Protonen abgeben können. insbesondere weist der erste Wirkstoff keine sauren Gruppen auf mit einem pK_{A}-Wert unter 5. Saure Gruppen umfassen insbesondere Carboxylgruppen, schwefelhaltige Säuregruppen wie Sulfonsäuregruppen und Schwefelsäureester, phosphorhaltige Säuregruppen, Hydroxylgruppen einschließlich phenolischer Hydroxylgruppen und Amidgruppen. insbesondere sind keine Carboxylgruppen und/oder Hydroxylgruppen im ersten Wirkstoff enthalten. Die Löslichkeit des ersten Wirkstoffs ist also besonders bevorzugt wesentlich von der basischen funktionellen Gruppe vorzugsweise ausgewählt aus Aminogruppe, Guanidingruppe und stickstoffhaltiger Heterozyklus, ganz besonders bevorzugt der wenigstens einen Aminogruppe, geprägt. Jedenfalls profitieren solche Wirkstoffe besonders vom Design der erfindungsgemäßen Arzneiform.

Der erste Wirkstoff weist bevorzugt bei pH-Werten < 7 und 22°C höhere Löslichkeiten auf als bei pH 7 und 22°C. Der erste Wirkstoff weist vorzugsweise bei pH 1 und 22°C in wässriger Lösung eine Löslichkeit von mehr als 0,1 mg/ml, weiter bevorzugt wenigstens 0,5 mg/ml, weiter bevorzugt wenigstens 1 mg/ml, noch weiter bevorzugt mindestens 1,3 mg/ml und besonders bevorzugt mindestens 1,5 mg/ml auf. Der Wirkstoff ist also bei einem pH Wert von 1 bevorzugt wenigstens "sehr schwer löslich", weiter bevorzugt wenigstens "schwer löslich" gemäß der Löslichkeitsklassifikation des Europäischen Arzneibuchs (Ph.Eur. 7). Bevorzugt liegt die Löslichkeit des ersten Wirkstoffs bei pH-Werten von mehr als 7 bei höchstens 0,01 mg/ml, weiter bevorzugt bei höchstens 0,001 mg/ml. Beispielsweise beträgt die Löslichkeit des Wirkstoffs bei pH 9 in wässriger Lösung und 22°C vorzugsweise höchstens 0,01 mg/ml und mehr bevorzugt höchstens 0,001 mg/ml.

Als wässrige Lösung zur Erzielung eines pH-Wertes von 1 wird die Salzsäure-Standardlösung des Europäischen Arzneibuchs (Ph. Eur. 7) verwendet aus Salzsäure und Wasser, das heißt eine 0,1 N (0,1 mol/l) Salzsäurelösung. Zur Ermittlung der Löslichkeit des Wirkstoffs bei pH 9 ist die Phosphat-Pufferlösung pH 9,0 R geeignet.

Der erfindungsgemäß eingesetzte erste Wirkstoff hat vorzugsweise einen n-Octanol-Wasser-Verteilungskoeffizienten (logP_{OW}) bei 20°C von mehr als 1, vorzugsweise mehr als 2, mehr bevorzugt mehr als 3 und besonders bevorzugt mehr als 4, insbesondere mehr als 5. Dieser Verteilungskoeffizient ist ein Maß für die Lipophilie einer Substanz. Wie zuvor erwähnt, profitieren besonders lipophile Wirkstoffe von der Arzneiform dieser Erfindung. Der Vorteil für solche Wirkstoffe ist auch deswegen besonders hoch, weil üblicherweise die Resorption lipophiler Wirkstoffe sehr gut funktioniert, sobald diese Substanzen gelöst sind. Mit anderen Worten ist die Auflösung dieser Wirkstoffe aus einer Arzneiform der geschwindigkeitsbestimmende Schritt bei der Aufnahme des Wirkstoffes in den Körper.

Trotzdem gibt es auch Wirkstoffe, deren Lipophilie so ausgeprägt ist, dass die hier vorgeschlagene Formulierung nicht ausreicht, um eine ausreichende Bioverfügbarkeit sicher zu stellen. Daher weist der erste Wirkstoff einen n-Octanol-Wasser-Verteilungskoeffizienten (logP_{OW}) bei 20°C von vorzugsweise höchstens 100, weiter bevorzugt höchstens 50, insbesondere höchstens 30, besonders bevorzugt höchstens 15 und insbesondere höchstens 7 auf.

Um die Löslichkeit des ersten Wirkstoffes nach Applikation weiter zu verbessern, ist es erfindungsgemäß bevorzugt, den ersten Wirkstoff in kleiner Partikelgröße zu verwenden. Daher weist der erste Wirkstoff in der Arzneiform dieser Erfindung vorzugsweise mittlere Partikelgrößen von höchstens 1000 nm, weiter bevorzugt höchstens 600 nm und besonders bevorzugt höchstens 300 nm auf. Die mittlere Partikelgröße wird erfindungsgemäß bevorzugt mit der Analysemethode der dynamischen Lichtstreuung ermittelt. Die kleine Partikelgröße ist insbesondere dann von Bedeutung, wenn sich der Wirkstoff im Emulgator nicht oder kaum löst. Dies trifft meist zu, wenn der Emulgator einen besonders großen hydrophilen Anteil hat, wie etwa viele Poloxamere, und/oder der erste Wirkstoff besonders lipophil ist. Anspruchsgemäß werden andere Emulgatoren als Poloxamere eingesetzt.

Es ist erfindungsgemäß bevorzugt, dass sich der erste Wirkstoff im Emulgator nahezu vollständig, besonders bevorzugt vollständig, löst. Dadurch wird eine verbesserte Freisetzung erreicht. Erzielt werden kann dieser Effekt dadurch, dass der lipophile Anteil des Emulgators an die Lipophilie des Wirkstoffes angepasst wird. Wie das erreicht werden kann, ergibt sich aus den weiter unten folgenden Ausführungen zum Emulgator und dessen Molekülstruktur. Der erste Wirkstoff liegt also besonders bevorzugt vollständig gelöst in der erfindungsgemäßen Arzneiform vor.

insbesondere Wirkstoffe, die bei Verwendung sofortfreisetzender Arzneiformen wenigstens dreimal täglich verabreicht werden müssen, profitieren von der erfindungsgemä-βen Arzneiform. Der erste Wirkstoff ist also vorzugsweise ein solcher, der bei Einnahme sofortfreisetzender Arzneiformen mindestens 3-mal täglich verabreicht werden muss.

Der erste Wirkstoff kann ausgewählt sein aus Wirkstoffen zur Behandlung von Schmerzen und zur Schmerztherapie, zur Behandlung von Erkrankungen des Nervensystems, zur Behandlung von psychiatrischen Erkrankungen, zur Behandlung von Herz-Kreislauf-Krankheiten, zur Behandlung der Migräne, zur Behandlung von Erkrankungen der Atemwege und der Lunge, zur Behandlung von Erkrankungen des Magen-Darm-Traktes und der Bauchspeicheldrüse, zur Infektabwehr einschließlich zur Behandlung von Pilzerkrankungen, zur Behandlung der erektilen Dysfunktion, zur Hormontherapie, zur Behandlung von allergischen Reaktionen und zur Behandlung von Schwindel jedweder Genese.

Besonders bevorzugt ist der erste Wirkstoff ausgewählt aus Wirkstoffen der Wirkstoffklassen Antihistaminika, Antiemetika, Antivertiginosa und/oder Calciumkanalblocker. in einer besonders bevorzugten Ausführungsform ist der erste Wirkstoff Cinnarizin, ein Cinnarizinsalz und/oder ein Cinnarizinderivat, insbesondere Prodrug. Ganz besonders bevorzugt ist der erste Wirkstoff Cinnarizin.

Der Anteil des ersten Wirkstoffs an der Arzneiform beträgt vorzugsweise wenigstens 1 Gew.-%, weiter bevorzugt wenigstens 2 Gew.-%, mehr bevorzugt wenigstens 3 Gew.-% und besonders bevorzugt wenigstens 5 Gew.-% bezogen auf die Gesamtmasse der erfindungsgemäßen Arzneiform. Ein gewisser Wirkstoffanteil ist notwendig, um die gewünschte pharmakologische Wirkung zu erzielen. Der Wirkstoffanteil an erstem Wirkstoff darf nicht zu hoch sein, weil die Verbesserung der Wirkstoffauflösung nicht für beliebig hohe Wirkstoffanteile mit hinreichender Sicherheit erzielt werden kann. Daher ist der Wirkstoffgehalt des ersten Wirkstoffs an der Arzneiform in bevorzugten Ausführungsformen auf höchstens 25 Gew.-%, weiter bevorzugt höchstens 18 Gew.-%, mehr bevorzugt höchstens 15 Gew.-% und besonders bevorzugt höchstens 12 Gew.-% bezogen auf die Gesamtmasse der erfindungsgemäßen Arzneiform beschränkt.

Der erste Wirkstoff wird vorzugsweise in einer Menge von wenigstens 20 mg oder besonders bevorzugt wenigstens 30 mg in der erfindungsgemäßen Arzneiform eingesetzt. Die Menge an erstem Wirkstoff soll vorzugsweise einen Wert von 100 mg und besonders bevorzugt 80 mg in der erfindungsgemäßen Arzneiform nicht übersteigen.

Der zweite Wirkstoff weist eine vom ersten Wirkstoff deutlich abweichende Löslichkeit auf.

Grundsätzlich ist die Verarbeitung und Darbietung von Wirkstoffen mit voneinander abweichenden Löslichkeitseigenschaften problematisch. Es besteht beispielsweise die Gefahr, dass der besser lösliche Wirkstoff zu schnell aus der Arzneiform freigesetzt wird, während der schlechter lösliche Wirkstoff nicht oder nur unzureichend freigesetzt wird. Mit der erfindungsgemäßen Arzneiform ist es möglich, Wirkstoffe mit voneinander abweichenden Löslichkeitseigenschaften zu verarbeiten, selbst Wirkstoffe mit deutlich abweichenden Löslichkeitseigenschaften. Deutlich abweichende Löslichkeitseigenschaften liegen erfindungsgemäß vor, wenn der zweite Wirkstoff eine Löslichkeit hat, die bei pH 7 und 22°C in wässriger Lösung um wenigstens eine Zehnerpotenz höher liegt als die Löslichkeit des ersten Wirkstoffs bei pH 7 und 22°C in wässriger Lösung. Bevorzugt liegt die Löslichkeit des zweiten Wirkstoffs bei pH 7 und 22°C sogar um den Faktor 50 höher als die Löslichkeit des ersten Wirkstoffs, ganz besonders bevorzugt sogar um den Faktor 100, also zwei Zehnerpotenzen. Solche Wirkstoffe profitieren besonders von der erfindungsgemäßen Formulierung, weil ihre Verarbeitung zusammen mit gering löslichen Wirkstoffen in bisher bekannten Arzneiformen an Grenzen stößt.

Der zweite Wirkstoff ist vorzugsweise ein Wirkstoff, der wenigstens eine Hydroxylgruppe, wenigstens eine Carboxylgruppe und/oder wenigstens eine permanente Ladung aufweist, vorzugsweise wenigstens eine permanente Ladung.

insbesondere solche zweiten Wirkstoffe mit einer hohen Verweildauer im Körper, ausgedrückt in einer hohen Eliminationshalbwertszeit profitieren von der erfindungsgemäßen Arzneiform. Denn gerade bei solchen Wirkstoffen ist es besonders wichtig, eine zu schnelle Freisetzung beispielsweise bei längerer Verweilzeit im Magen zu verhindern. Eine hohe Eliminationshalbwertszeit bedeutet erfindungsgemäß eine Eliminationshalbwertszeit von mehr als 2 Stunden, bevorzugt von mehr als 2,5 Stunden, weiter bevorzugt von wenigstens 3 Stunden.

Grundsätzlich kann jeder zweite Wirkstoff mit der oben angegebenen Löslichkeit von der Darreichung in der erfindungsgemäßen Arzneiform profitieren, der über einen längeren Zeitraum verlängert verabreicht werden soll. Beispiele für Wirkstoffe und Wirkstoffklassen, welche vorteilhaft als zweiter Wirkstoff im Rahmen der vorliegenden Erfindung eingesetzt werden können, sind:
Arzneimittel zur Behandlung von Schmerzen und zur Schmerztherapie mit peripher wirkenden Analgetika, zentral wirkenden Analgetika und adjuvante Nicht-Analgetika. Es handelt sich hier vorzugsweise um folgende Analgetika und adjuvante Stoffe:
Paracetamol, Metamizol, Celecoxib, Parecoxib, Tramadol, Pethidin, Codein, Dihydrocodein, Piritramid, Tilidin, Morphin, Hydromorphon, Oxycodon, Levomethadon, Fentanyl, Sufentanil, Buprenorphin, Pentazocin, Naloxon, Flupirtin, Carbamazepin, Metoprolol, Metoclopramid, Amitryptilin, Doxepin, Clomipramin, Mianserin, Maprotilin, Triptane wie z.B. Naratriptan, Rizatriptan, Sumatriptan, Zolmitriptan, Calciumantagonisten wie: Flunarizin, Topiramat, Valproinsäure, Phenytoin, Baclofen, sonstige Mittel wie: Botulinum Toxin, Ergotamine, Lisurid, Methysergid, Pizotifen, Oxcarbazepin, Gabapentin und Lamotrigin, Dexamethaon, Methylprednisolon, Prednisolon, Triamcinolon, Diazepam, Tetrazepam, Tizanidin, Butylscopolamin und/oder Kombination von Tilidin und Naloxon.

Arzneistoffe zur Behandlung des Nervensystems, allein oder in Kombination, z.B. Anfallsleiden (insbesondere Clonazepam, Diazepam, Lorazepam, Midazolam, Clobazam, Phenytoin, Clomethiazol, Valproinsäure, Phenobarbital, Gabapentin, Lamotrigin, Oxcarbazepin, Pregabalin, Topiramat, Ethosuximid, Levetrazetam, Mesuximid, Primidon, Nitrazepam und/oder Vigabitrin), Parkinson-Syndrom (insbesondere Levodopa, mit Benserazid/Carbidopa, Bromocriptin, Cabergolin, Dihydroergocriptin, Lisurid, Pergolidmesilat, Pramipexol, Ropinirol, Apomorphin, Biperiden, Metixenhydrochlorid, Trihexphenidyl, Entacapon, Amantadin, Bupidin, Selegilin und/oder Apomorphin), Schlaganfall (insbesondere Clopidogrel, Dipyridamol, Ticlopidin, Heparin, Phenprocoumon, Warfarin, Protamin, Phytomenadion, Nimodipin, Paracetamol, Tramadol und/oder Buprenorphin), Hirndruck (insbesondere Furosemid und/oder Mannitol), Tremor (insbesondere Propranolol, Clozapin, Alprazolam und/oder Primidon).

Arzneistoffe zur Behandlung von psychiatrischen Erkrankungen wie Angststörungen (insbesondere Alprazolam, Diazepam, Fluoxetin, Paroxetin, Chlorprothixen, Levomepromazin, Thioridazin, Flupentixol und/oder Fluspirilen), Depressionen (insbesondere Imipramin, Amitripytlin, Desipramin, Maprotilin, Minaserin, Citalopram, Fluoxetin, Paroxetin, Trazodon, Moclobemid und/oder Mirtazapin), Psychosen und Schizophrenien (insbesondere Sulpirid, Promazin, Melperon, Thioridazin, Chlorprothixen, Perazin, Pimozid, Fluphenazin, Olanzapin und/oder Risperidon), Schlafstörungen (insbesondere Triazolam, Brotizolam, Oxazepam, Flurazepam, Nitrazepam, Temazepam, Zolpidemtartrat, Zopiclon, Promethazin, Chlorprothixen, Pipamperon, Thioridazin und/oder Chloralhydrat) Unruhezustände und Verwirrtheit (insbesondere Alprazolam, Oxazepam, Doxepin, Clomipramin, Imipramin, Thioridazin und/oder Perazin), Demenz vom Alzheimer-Typ (insbesondere Donepezil, Rivastigmin, Tacrin, Memantin, Nimodipin und/oder Seleginin).

Arzneistoffe zur Behandlung von Herz-Kreislauf-Krankheiten, wie koronare Herzkrankheit/Angina Pectoris (insbesondere Clopidrogel, Ticlopidin, Isosorbiddinitrat, Isosorbidmononitrat, Nitroglycerin, Molsidomin, Trapidil, Metoprolol, Bisoprolol, Atenolol, Acebutolol, Carvedilol, Nitrendipin, Nifedipin, Diltiazem, Verapamil, Benazepril, Lisinopril, Ramipril, Fosinopril und/oder Enalapril), Herzinfarkt und Herzinsuffizienz (insbesondere Isosorbidmono- und dinitrat, Clopidogrel, Ticlopidin, Captoprol, Ramipril, Lisinopril, Candesartan, Eproartan, Irbesatan, Losartan, Chlortalidon, Xipamid, Hydrochlorothiazid, Furosemid, Piretanid, Triameteren, Digitalisglycoside, Carvedilol, Metoprolol und/oder Prazosin), Herzrhythmusstörungen (insbesondere Ajmalin, Chinidin, Disopyramid, Flecainid, Propafenon, Propranolol, Carvedilol, Amiodaron, Verapamil und/oder Diltiazem), Hypertonie (insbesondere Metoprolol, Atenolol, Urapidil, Clonidin, Dihydralazin, Chlortalidon, Hydrochlorothiazid, Furosemid, Felodipin, Israpidin, Lacidipin, Diltiazem, Captopril, Enalapril, Fosinopril, Lisinopril, Ramnipril, Verapamil, Candesartan, Eprosartan, Irbesatan, Losartan, Doxazosin, Bunazosin, Prazosin, Terazosin, Moxonidin, Dihydralazin und/oder Minoxidil).

Arzneistoffe, allein oder in Kombination, zur Behandlung von Erkrankungen der Atemwege und der Lunge (insbesondere Theophyllin, Methylprednisolon, Flucorton, Dexamethason, Montekulast, Roxithromycin, Erythromycin, Azithromycin, Ciprofloxacin, Clarithromycin, Levofloxacin, Ofloxacin, Doxycyclin, Ampicillin und Sulbactam, Amoxicillin, Cefuroxim, Clindamycin, Cefotiam, Cefuroxim, Ceftazidim, Ceftriaxon, Piperacillin und/oder Moxifloxacin).

Arzneistoffe zur Behandlung der erektilen Dysfunktion (insbesondere Sildenafil, Tadalafil, Vardenafil, Theobromin, Koffein und/oder Theophyllin).

Vorzugsweise ist der zweite Wirkstoff ein solcher, der zusammen mit dem ersten Wirkstoff eine synergistische Wirkung zeigt. Dies bedeutet erfindungsgemäß, dass die Wirkung von erstem und zweitem Wirkstoff bei Einnahme der erfindungsgemäßen Arzneiform verstärkt ist im Vergleich zur Einzelanwendung von erstem oder zweitem Wirkstoff. Gerade bei solchen synergistisch wirkenden Wirkstoffen ist es wichtig, dass die Freisetzungsprofile beider Wirkstoffe aufeinander abgestimmt sind oder werden. Nur so ist auch der synergistische Effekt während der ganzen Zeit vorhanden. Gerade für synergistische Wirkstoffe bietet diese Erfindung also eine deutliche Verbesserung gegenüber dem Stand der Technik. Ganz besonders bevorzugt gehören erster und zweiter Wirkstoff derselben Wirkstoffklasse an.

Ganz besonders bevorzugt ist der zweite Wirkstoff ausgewählt aus der Wirkstoffklasse Antihistaminika, Antiemetika, Calciumkanalblocker und/oder Antivertiginosa. Ganz besonders bevorzugt ist der zweite Wirkstoff Dimenhydrinat.

Der zweite Wirkstoff ist bevorzugt in Form kleiner Partikel in der erfindungsgemäßen Arzneiform enthalten. Das bedeutet, dass die mittlere Partikelgröße des zweiten Wirkstoffes vorzugsweise 1000 nm nicht übersteigt. Weiter bevorzugt beträgt die mittlere Partikelgröße des zweiten Wirkstoffes nicht mehr als 300 nm. Die Partikelgröße des Wirkstoffes wird mittels Laserdiffraktion bestimmt.

Der Anteil des zweiten Wirkstoffes an der Arzneiform beträgt vorzugsweise wenigstens 4 Gew.-%, weiter bevorzugt wenigstens 8 Gew.-% bezogen auf die Gesamtmasse der erfindungsgemäßen Arzneiform. Der Wirkstoffanteil an zweitem Wirkstoff darf nicht zu hoch sein, weil die Modifikation der Freisetzung nicht für beliebig hohe Wirkstoffanteile mit hinreichender Sicherheit erzielt werden kann. Daher ist der Wirkstoffgehalt des zweiten Wirkstoffs an der Arzneiform in bevorzugten Ausführungsformen auf höchstens 30 Gew.-%, weiter bevorzugt höchstens 18 Gew.-% beschränkt.

in bevorzugten Ausführungsformen umfasst die erfindungsgemäße Arzneiform keine Antibiotika in pharmazeutisch wirksamer Menge, insbesondere keinen Vertreter der Wirkstoffklasse der Tetracycline. Antibiotika beeinträchtigen die Darmflora, in dem sie wichtige Bakterien im Darm abtöten. in der Arzneiform dieser Erfindung würde dieser Effekt zu starken Nebenwirkungen führen, weil die Arzneiform sich teilweise erst sehr spät auflöst.

in bevorzugten Ausführungsformen umfasst die erfindungsgemäße Arzneiform keine Protonenpumpenhemmer. Protonenpumpenhemmer erhöhen den pH-Wert im Magen und stören somit die Auflösung vieler Arzneistoffe, insbesondere schwach basischer Wirkstoffe.

in bevorzugten Ausführungsformen umfasst die erfindungsgemäße Arzneiform keine nichtsteroidalen Antirheumatika (NSAR), also sind erster und zweiter Wirkstoff sowie optional die weiteren Wirkstoffe bevorzugt nicht ausgewählt aus Wirkstoffen aus der Gruppe der nichtsteroidalen Antirheumatika. Nichtsteroidale Antirheumatika können zu einer Steigerung der Magensäureproduktion und einer Verminderung der Magenschleimbildung führen, was mit einer Schädigung der Magen- und Darmschleimhaut einhergehen kann. Dies kann die Resorption des in der Arzneiform enthaltenen Wirkstoffanteils nachteilig beeinflussen.

in der erfindungsgemäßen Arzneiform können weitere Wirkstoffe enthalten sein neben dem ersten und dem zweiten Wirkstoff. Ganz besonders bevorzugt besteht der Wirkstoffanteil der Arzneiform aber aus dem ersten und dem zweiten Wirkstoff.

Der Gesamtwirkstoffgehalt der Arzneiform, vorzugsweise bestehend aus dem ersten und dem zweiten Wirkstoff, beträgt dabei bevorzugt höchstens 50 Gew.-%, weiter bevorzugt höchstens 30 Gew.-% und besonders bevorzugt höchstens 28 Gew.-% bezogen auf die Gesamtmasse der erfindungsgemäßen Arzneiform. Bei zu hohem Gesamtwirkstoffgehalt der Arzneiform kann die Steigerung der Auflösung bzw. die Modifikation der Freisetzung der Wirkstoffe nicht mehr optimal erfolgen, beziehungsweise würde die Arzneiform, um eine optimale Freisetzung sicherzustellen, insgesamt zu groß und damit schwerer schluckbar.

Erster und/oder zweiter Wirkstoff können einen unangenehmen Geschmack aufweisen, beispielsweise einen bitteren und/oder betäubenden Geschmack. insbesondere solche ersten und/oder zweiten Wirkstoffe profitieren von den erfindungsgemäßen Arzneiformen. Die erfindungsgemäße Zusammensetzung der Arzneiformen ermöglicht nämlich eine optimale Maskierung eines unangenehmen Geschmacks des ersten und/oder zweiten Wirkstoffs. Ebenso kann erster und/oder zweiter Wirkstoff begrenzt lagerstabil sein. Auch begrenzt lagerstabile Wirkstoffe können mit den erfindungsgemäßen Arzneiformen optimal und in einer lagerstabilen Form dargeboten werden.

Der Emulgator verbessert die Auflösung des ersten Wirkstoffs und trägt gleichzeitig zur Modifikation der Freisetzung des ersten und des zweiten Wirkstoffs bei. Dies ist besonders wichtig, damit sich wenigstens ein Teil des ersten Wirkstoffs möglichst zügig und gleichmäßig bereits im Magen auflöst. Wie oben beschrieben, sinkt der pH-Wert des Magens nach Einnahme einer Mahlzeit nur langsam wieder. Es ist aber vorteilhaft, dass sich der erste Wirkstoff bereits jetzt im Magen löst, damit er mit dem Speisebrei vermischt wird. Das Vermischen erfolgt durch die Magenbewegung.

Bevorzugt liegt der erste Wirkstoff eingelagert in eine Emulgatormatrix vor, und ist besonders bevorzugt darin gelöst. Dadurch wird die Oberfläche des ersten Wirkstoffs vergrößert. Gleichzeitig löst sich der Emulgator vorzugsweise nur langsam im Magen-Darm-Trakt und ist dadurch in der Lage, die Freisetzung von erstem und zweitem Wirkstoff zu modifizieren.

Damit die Verbesserung der Auflösung und Modifikation der Freisetzung des Wirkstoffanteils besonders gut ausgeprägt ist, soll der Emulgator vorzugsweise einen HLB-Wert von wenigstens 1 und höchstens 16, insbesondere wenigstens 9 und höchstens 14 aufweisen. Der Emulgator ist vorzugsweise nichtionisch.

Es hat sich gezeigt, dass solche Emulgatoren besonders vorteilhafte Ergebnisse erzielen, die ein bestimmtes Strukturmotiv aufweisen. Dieses Strukturmotiv ist eine Polyethylenglykolkette. Es wurde gefunden, dass Emulgatoren mit diesem Strukturmotiv, also wenigstens einer Polyethylenglykolkette, eine Doppelfunktion besitzen. Einerseits führen sie bei hohen pH-Werten ab etwa pH 4 zu einer Verbesserung der Löslichkeit des ersten Wirkstoffes. Andererseits führen sie bei niedrigen pH-Werten, nämlich dann, wenn der erste Wirkstoff vorzugsweise eine höhere Löslichkeit aufweist, zu einer Verzögerung der Freisetzung. Dies ist ein erfindungsgemäß erwünschtes Verhalten, soll doch die Freisetzung des ersten Wirkstoffes möglichst kontinuierlich über einen langen Zeitraum erfolgen.

Die Polyethylenglykolkette stellt den hydrophilen Teil des Emulgators dar und ist mit einem lipophilen Teil verbunden. Der lipophile Teil kann vorteilhafterweise ein Glyceridrest oder eine andere Polyalkylenoxidkette sein.

Als Glyceridreste kommen Mono- und Diglyceridreste in Frage, wobei Diglyceridreste bevorzugt sind. Monoglyceridreste sind unter Umständen, je nach Kettenlänge der beteiligten Fettsäurereste nicht lipophil genug.

Als Polyalkylenoxidketten kommen insbesondere Polyalkylenoxide in Frage, die ununterbrochene Kohlenstoffketten von wenigstens 3 Kohlenstoffatomen aufweisen. Ein bevorzugtes Beispiel für eine Polyalkylenoxidkette im Emulgator dieser Erfindung ist Polypropylenoxid.

Diese Emulgatoren können mit dem Fachmann bekannten Verfahren erhalten werden. Polyethylenglykolglyceride können beispielsweise durch Reaktion von den entsprechenden Glyceriden mit Polyethylenglykol hergestellt werden. Kommerziell erhältliche Polyethylenglykolglyceride sind beispielsweise Gelucire^{®} 43/01 und Gelucire^{®} 50/13.

Mit anderen Worten: die Emulgatoren, die in der erfindungsgemäßen Arzneiform eingesetzt werden, weisen folgendes Strukturelement auf:

R¹-O-[CH₂-CH₂-O]ₙ-R² (Formel I)

Darin sind R¹ und R² gleich oder verschieden. R¹ und R² sind unabhängig voneinander Wasserstoff, Alkyl, Glycerid oder Polyalkylenoxid. Alkyl ist bevorzugt kurzkettig, d.h. es weist eine Kettenlänge von höchstens 6 Kohlenstoffatomen auf. Bevorzugt sind R¹ und R² unabhängig voneinander Wasserstoff, Glycerid oder Polyalkylenoxid.

Wenn ein Rest R¹ oder R² Wasserstoff ist, sind die Reste R¹ und R² verschieden, d.h. der jeweils andere Rest ist nicht Wasserstoff; und wenn R¹ oder R² Alkyl ist, sind die Reste R¹ und R² verschieden, d.h. der jeweils andere Rest ist nicht Alkyl. Andernfalls hätte das Molekül nicht die erforderliche emulgierende Wirkung. Vorzugsweise sind R¹ und R² weder Wasserstoff noch Alkyl, wenn der jeweils andere Rest Wasserstoff oder Alkyl ist.

n ist die Anzahl der Kettenglieder in der Polyethylenoxidkette. n ist eine ganze Zahl von wenigstens 4, bevorzugt wenigstens 10 und besonders bevorzugt wenigstens 20. ist n kleiner, so ist der erfindungsgemäß erwünschte Effekt nicht sehr stark ausgeprägt, weil der lipophile Teil des Emulgators zu klein ist. in bevorzugten Ausführungsformen ist n nicht größer als 100, insbesondere nicht größer als 50, weiter bevorzugt nicht größer als 40 oder nicht größer als 30. Es hat sich gezeigt, dass größere Kettenlängen dazu führen, dass der Emulgator vom Pankreatin und Lipasen langsamer abgebaut wird. Dadurch lässt sich die verlängerte Freisetzung steuern. ist die Kette aber zu lang, setzt die Arzneiform den ersten Wirkstoff nicht schnell genug frei.

Wenn R¹ und/oder R² ein Polyalkylenoxidrest sind, so hat der Rest folgende allgemeine Formel:

-O-[Y-O]ₘ-R³ (Formel II)

Darin ist R³ Wasserstoff oder Alkyl, insbesondere kurzkettiges Alkyl (bis C₆). Y ist eine Alkylengruppe mit einer Kohlenstoffkettenlänge von wenigstens C₃ und vorzugsweise höchstens C₆, weiter bevorzugt höchstens C₄.

m bezeichnet die Anzahl der Kettenglieder in der Polyalkylenoxidkette. m ist vorzugsweise eine ganze Zahl von wenigstens 3, insbesondere wenigstens 5 und besonders bevorzugt wenigstens 10. m soll vorzugsweise eine ganze Zahl von 50, insbesondere 40 und besonders bevorzugt 30 oder 20 nicht überschreiten. in bevorzugten Ausführungsformen sind R¹ und R² Polyalkylenoxidreste.

Wenn R¹ und/oder R² Glyceridreste sind, so hat der Glyceridrest folgende allgemeine Formel:

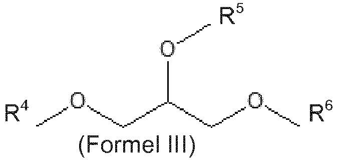

Dabei ist die Formel III an einer der Stellen R⁴, R⁵ oder R⁶ mit dem Strukturelement der Formel I verknüpft. Es ist erfindungsgemäß unerheblich, an welcher der Stellen die Struktur der Formel III mit der Struktur der Formel I verknüpft ist. Es ist also einer der Reste R⁴, R⁵ oder R⁶ die Struktur der Formel I. Für die übrigen beiden Reste gilt dann folgendes:
R⁴ ist vorzugsweise Wasserstoff oder ein Fettsäurerest. Der Fettsäurerest R⁴ weist eine Kettenlänge von vorzugsweise wenigstens C₆, weiter bevorzugt wenigstens C₈ und besonders bevorzugt wenigstens C₁₀ auf. Eine Mindestkettenlänge sollte eingehalten werden damit der Glyceridrest hinreichend lipophil ist. Die Anzahl der Kohlenstoffatome in den Fettsäuren ist vorzugsweise gradzahlig. Die Kettenlänge soll vorzugsweise einen Wert von C₂₂, weiter bevorzugt C₁₈ und besonders bevorzugt C₁₆ nicht überschreiten, weil andernfalls die Löslichkeit des Emulgators beeinträchtigt werden könnte.

R⁵ ist vorzugsweise Wasserstoff oder ein Fettsäurerest. Der Fettsäurerest R⁵ weist eine Kettenlänge von vorzugsweise wenigstens C₆, weiter bevorzugt wenigstens C₈ und besonders bevorzugt wenigstens C₁₀ auf. Eine Mindestkettenlänge sollte eingehalten werden damit der Glyceridrest hinreichend lipophil ist. Die Anzahl der Kohlenstoffatome in den Fettsäuren ist vorzugsweise gradzahlig. Die Kettenlänge soll vorzugsweise einen Wert von C₂₂, weiter bevorzugt C₁₈ und besonders bevorzugt C₁₆ nicht überschreiten, weil andernfalls die Löslichkeit des Emulgators beeinträchtigt werden könnte.

R⁶ ist vorzugsweise Wasserstoff oder ein Fettsäurerest. Der Fettsäurerest R⁶ weist eine Kettenlänge von vorzugsweise wenigstens C₆, weiter bevorzugt wenigstens C₈ und besonders bevorzugt wenigstens C₁₀ auf. Eine Mindestkettenlänge sollte eingehalten werden damit der Glyceridrest hinreichend lipophil ist. Die Anzahl der Kohlenstoffatome in den Fettsäuren ist vorzugsweise gradzahlig. Die Kettenlänge soll vorzugsweise einen Wert von C₂₂, weiter bevorzugt C₁₈ und besonders bevorzugt C₁₆ nicht überschreiten, weil andernfalls die Löslichkeit des Emulgators beeinträchtigt werden könnte. Vorzugsweise sind zwei der Reste R⁴, R⁵ und R⁶ Fettsäurereste. Entsprechend handelt es sich dann um eine Diglyceridgruppe.

Es ist zu beachten, dass die richtige Kettenlänge der Fettsäurereste stark mit der Anzahl der Kettenglieder in der Polyethylenoxidkette (n) korrespondiert. ist die Polyethylenoxidkette länger, können auch die Fettsäureketten länger sein.

Die hier beschriebenen Emulgatoren lassen sich durch Reaktion von entsprechenden Mono-, Di- und/oder Triglyceriden mit entsprechenden Polyalkylenoxiden herstellen. Diese Edukte sind kommerziell erhältlich. Die erfindungsgemäßen Emulgatoren sind vorzugsweise Mischungen der genannten Substanzen.

in bevorzugten Ausführungsformen ist der Emulgator ausgewählt aus Gelucire^{®} 50/13, Gelucire^{®} 43/01 und Mischungen daraus, ganz besonders bevorzugt ist Gelucire^{®} 50/13.

Eine gute Lösungsvermittlung wird erzielt, wenn das Verhältnis der Massen von erstem Wirkstoff zu Emulgator wenigstens 1 zu 30, weiter bevorzugt wenigstens 1 zu 20 und besonders bevorzugt wenigstens 1 zu 10 beträgt. Das genannte Verhältnis beträgt vorzugsweise höchstens 1 zu 1, weiter bevorzugt höchstens 1 zu 2 und besonders bevorzugt höchstens 1 zu 3. Grundsätzlich gilt, dass eine größere Menge an Emulgator eine bessere Lösungsvermittlung ermöglicht. Außerdem trägt der Emulgator vorzugsweise auch zur Modifikation der Freisetzung des zweiten Wirkstoffs bei. Es muss aber berücksichtigt werden, dass eine Arzneiform bestimmte Höchstvolumina nicht überschreiten sollte, damit die Einnahme nicht unnötig unangenehm ist. Außerdem kann eine zu große Menge an Emulgator auch dazu führen, dass die Auflösung und/oder die Freisetzung des ersten Wirkstoffes behindert werden.

Ein Masseverhältnis von zweitem Wirkstoff zu Emulgator von wenigstens 1:20, bevorzugt mindestens 1:10 und besonders bevorzugt mindestens 1:5 ist vorteilhaft, da der Emulgator auch zur Modifikation des zweiten Wirkstoffs beitragen soll. Bei zu hohen Anteilen an Emulgator im Verhältnis zum zweiten Wirkstoff besteht die Gefahr, dass die Freisetzung des zweiten Wirkstoffs behindert und die Arzneiform insgesamt zu groß wird. Das Masseverhältnis von zweitem Wirkstoff zu Emulgator sollte allerdings einen Wert von 1:1,2 und bevorzugt 1:1,5 nicht überschreiten. Bei einem zu geringen Anteil von Emulgator im Verhältnis zu zweitem Wirkstoff kann der Emulgator nicht mehr ausreichend zur Modifikation der Freisetzung des zweiten Wirkstoffs beitragen.

Die Arzneiform enthält den Emulgator vorzugsweise in einem Anteil von wenigstens 15 Gew.-%, weiter bevorzugt wenigstens 20 Gew.-%, noch weiter bevorzugt wenigstens 22 Gew.-% und besonders bevorzugt wenigstens 25 Gew.-% bezogen auf die Gesamtmasse der Arzneiform. Der Anteil des Emulgators soll vorzugsweise einen Anteil von 50 Gew.-%, weiter bevorzugt 40 Gew.-% und besonders bevorzugt 35 Gew.-% bezogen auf die Gesamtmasse der Arzneiform nicht überschreiten. Beim Versuch, das optimale Verhältnis von Lösungsvermittlung und Volumen der Arzneiform zu ermitteln, haben sich diese Mengen als vorteilhaft erwiesen.

in besonders bevorzugten Ausführungsformen wird zusätzlich zu den oben genannten Substanzen mindestens ein Phospholipid eingesetzt. Das Phospholipid ist vorzugsweise ausgewählt aus Phosphatidylcholinen (Lecithine), Phosphatidylethanolaminen (Kephaline), Phosphatidylserinen, Sphingomyelinen und Mischungen davon. Besonders bevorzugt sind Phosphatidylcholine (Lecithine). Es wurde überraschend gefunden, das Phospholipide ebenfalls die oben beschriebenen vorteilhaften Wirkungen hervorrufen, wie die oben genannten Emulgatoren.

Vorzugweise umfasst der Emulgator das Phospholipid zusätzlich zu mindestens einer der oben genannten Substanzen. Das Phospholipid verstärkt dabei die vorteilhaften Wirkungen der oben genannten Substanzen.

Die Arzneiform dieser Erfindung umfasst wenigstens ein Retardierungsmittel. Dieses dient der Modifikation der Freisetzung wenigstens des zweiten Wirkstoffs, weiter bevorzugt hat das Retardierungsmittel keinen messbaren Einfluss auf die Freisetzung des ersten Wirkstoffs.

Geeignete Retardierungsmittel können anorganischer oder organischer Art sein. Das Retardierungsmittel ist vorzugsweise ausgewählt aus einem natürlichen Polymer, einem synthetischen Polymer und Mischungen daraus.

Besonders bevorzugt ist das Retardierungsmittel in Wasser quellfähig, also in der Lage, durch Wasseraufnahme an Volumen zu gewinnen und somit eine Diffusionsbarriere für den zweiten Wirkstoff darzustellen. Bevorzugte Retardierungsmittel haben ein Quellvermögen von mehr als 1,5, bevorzugt mehr als 3, weiter bevorzugt mehr als 5. Es ist vorteilhaft, wenn das Retardierungsmittel ein Quellvermögen von höchstens 20 aufweist, bevorzugt höchstens 15. Dies bedeutet, dass das Volumen des gequollenen Retardierungsmittels vorzugsweise nicht mehr als das 20-fache, bevorzugt nicht mehr als das 15-fache des Volumens des nicht gequollenen Retardierungsmittels beträgt. Ein zu hohes Quellvermögen des Retardierungsmittels kann, da dieses vorzugsweise in einem verhältnismäßig hohen Anteil an der Arzneiform enthalten ist, die Freisetzung des zweiten Wirkstoffs behindern. Das Quellvermögen kann mit dem Fachmann bekannten Verfahren ermittelt werden, beispielsweise durch mikroskopische Verfolgung der Volumenzunahme bei der Quellung unter jeweils geeigneten Bedingungen zur Initiierung einer Quellung.

Bevorzugt wird ein Retardierungsmittel verwendet, dass in einer wässrigen Lösung in einem Anteil von 2 Gew.-% bei einer Temperatur von 20°C und einem Druck von 101,325 kPa eine Viskosität von wenigstens 500 mPas aufweist. Die Viskosität wird mit einem Kapillarviskosimeter (DIN 53015) gemessen. Je nach gewünschter Verlängerung der Freisetzung des zweiten Wirkstoffes kann es erwünscht sein, auch höhere Viskositäten zu wählen. Je höher die Viskosität des Retardierungsmittels, desto stärker wird die Modifikation der Freisetzung ausfallen. in besonders bevorzugten Ausführungsformen beträgt die Viskosität des Retardierungsmittels sogar wenigstens 2500 mPas und besonders bevorzugt wenigstens 3500 mPas. ist die Viskosität allerdings zu hoch, wird der zweite Wirkstoff zu langsam freigesetzt. Dies würde dazu führen, dass der zweite Wirkstoff während seiner Passage durch den Magen-DarmTrakt unter Umständen nicht vollständig freigesetzt wird oder erst in unteren Darmabschnitten, so dass therapeutische Plasmaspiegel nicht erzielt werden könnten. Die Viskosität ist vorzugsweise auf höchstens 200 000 mPas, weiter bevorzugt auf höchstens 120 000 mPas, noch weiter bevorzugt auf höchstens 90 000 mPas und am meisten bevorzugt auf höchstens 15 000 mPas beschränkt.

Bevorzugte Retardierungsmittel weisen ein mittleres Molekulargewicht (Zahlenmittel M_{N}) von wenigstens 5.000, weiter bevorzugt wenigstens 12.000 und besonders bevorzugt wenigstens 20.000 auf. Retardierungsmittel mit zu kleinen mittleren Molekulargewichten weisen oft nicht die nötigen Festigkeiten auf, um stabile und abriebfeste Arzneiformen herstellen zu können. Retardierungsmittel mit zu hohen mittleren Molekulargewichten hingegen lösen sich oft schlechter auf, so dass die Freisetzung des zweiten Wirkstoffs behindert werden kann und die Gefahr besteht, dass dieser erst in tiefen Darmabschnitten freigesetzt wird und somit nicht mehr ausreichend resorbiert werden kann. Das Retardierungsmittel hat vorzugsweise ein mittleres Molekulargewicht von höchstens 250.000, weiter bevorzugt höchstens 200.000 und am meisten bevorzugt höchstens 150.000.

Das Retardierungsmittel ist vorzugsweise ausgewählt aus natürlichen oder synthetischen Polysacchariden, Polymethacrylaten und deren Copolymeren, Polyacrylaten und Mischungen daraus.

Besonders bevorzugt umfasst das Retardierungsmittel ein natürliches oder synthetisches Polysaccharid. Besonders bevorzugt besteht das Retardierungsmittel aus einem natürlichen oder synthetischen Polysaccharid. Bevorzugte Polysaccharide weisen mehr als 10 Monosaccharidbausteine auf. Als geeignete Monosaccharidbausteine haben sich Pentosen und Hexosen erwiesen, weiter bevorzugt ausgewählt aus Glucose, Galactose, Xylose, Fructose, Arabinose, Mannose, Mannuronsäure, Guluronsäure, Gulose und Mischungen daraus.

Besonders bevorzugte natürliche oder synthetische Polysaccharide sind ausgewählt aus Cellulosen, Cellulosederivaten, Alginaten und Mischungen daraus.

Ganz besonders bevorzugt umfasst das Retardierungsmittel ein Cellulosederivat. Geeignete Cellulosederivate weisen durchschnittliche Substitutionsgrade von wenigstens 1 auf, weiter bevorzugt wenigstens 1,2 und am meisten bevorzugt wenigstens 1,3. Der Substitutionsgrad bezeichnet die mittlere Anzahl von Substituenten pro Glucoseeinheit der Cellulose. Bei zu geringem Substitutionsgrad können die Quellfähigkeit des Retardierungsmittels und die Viskosität vermindert sein, so dass die Freisetzung des zweiten Wirkstoffs nicht mehr ausreichend modifiziert wird.

Bevorzugte Cellulosederivate sind ausgewählt aus Methylcellulose, Hydroxypropylcellulose, Hydroxypropylmethylcellulose und Mischungen daraus. Besonders bevorzugte Cellulosederivate sind ausgewählt aus Methylcellulose, Hydroxypropylmethylcellulose und Mischungen daraus. Ein am meisten bevorzugtes Cellulosederivat ist Hydroxypropylmethylcellulose. Ganz besonders bevorzugt besteht das Retardierungsmittel aus Hydroxypropylmethylcellulose.

Als besonders geeignet als Retardierungsmittel haben sich hochviskose Hydroxypropylmethylcellulosen erwiesen mit einer Viskosität von mehr als 1.000 mPas. Vorzugsweise beträgt die Viskosität der Hydroxypropylmethylcellulose nicht mehr als 120.000 mPas, weiter bevorzugt nicht mehr als 20.000 mPas. Diese Werte haben sich erfindungsgemäß als besonders vorteilhaft erwiesen, um die Freisetzung des zweiten Wirkstoffs optimal zu modifizieren. Die Bestimmung der Viskosität erfolgt nach den oben beschriebenen Verfahren und Messbedingungen.

Besonders bevorzugt umfasst das Retardierungsmittel keine Polymere mit permanenter Ladung. Besonders bevorzugt weist das Retardierungsmittel im Wesentlichen keine ionischen Substanzen auf, insbesondere keine Natriumcarboxymethylcellulosen. Dies bedeutet erfindungsgemäß, dass das Retardierungsmittel weniger als 15 Gew.-%, bevorzugt weniger als 5 Gew.-% und weiter bevorzugt höchstens 1 Gew.-% Substanzen mit permanenter Ladung umfasst. Ganz besonders bevorzugt sind keine Substanzen mit permanenter Ladung im Retardierungsmittel enthalten. Sind nämlich Substanzen mit permanenten Ladungen im Retardierungsmittel in zu hoher Menge enthalten, so besteht die Gefahr, dass es zu Prezipitatbildung mit anderen Substanzen mit permanenter Ladung kommt, wie etwa dem zweiten Wirkstoff. Eine Prezipitatbildung kann für die Stabilität der Arzneiform und/oder die Freisetzung des zweiten Wirkstoffs nachteilig sein. Besonders bevorzugt enthält also das Retardierungsmittel und weiter bevorzugt die erfindungsgemäße Arzneiform keine Natriumcarboxymethylcellulosen.

Der Anteil des Retardierungsmittels an der Arzneiform soll vorzugsweise wenigstens 15 Gew.-%, weiter bevorzugt wenigstens 20 Gew.-%, noch weiter bevorzugt wenigstens 22 Gew.-% und besonders bevorzugt wenigstens 25 Gew.-% betragen. Wird eine zu geringe Menge des Retardierungsmittels eingesetzt, wird der gewünschte Effekt nicht erreicht. Bei zu großen Mengen wird der zweite Wirkstoff nicht schnell genug freigesetzt und die Verarbeitbarkeit der Arzneiform kann erheblich erschwert sein. Daher soll der Anteil an Retardierungsmittel an der Arzneiform einen Wert von 55 Gew.-%, weiter bevorzugt 45 Gew.-% und besonders bevorzugt 35 Gew.-% nicht übersteigen.

Ein wesentliches Merkmal der erfindungsgemäßen Arzneiformen ist es, dass die Summe der Gewichtsanteile an Emulgator und Retardierungsmittel wenigstens 40 Gew.-% bezogen auf die Gesamtmasse der Arzneiform beträgt. Sowohl Emulgator als auch Retardierungsmittel tragen zur Modifikation der Freisetzung des zweiten Wirkstoffs bei. Daher ist es wichtig, dass die Summe der Anteile wenigstens 40 Gew.-% beträgt, um die Freisetzung des zweiten Wirkstoffs optimal zu modifizieren und Plasmaspitzenspiegel durch eine zu schnelle Freisetzung insbesondere bei längerer Verweilzeit der Arzneiform im Magen effektiv zu verhindern. Gerade das synergistische Zusammenwirken von Emulgator und Retardierungsmittel ermöglicht dabei eine optimale Freisetzung des zweiten Wirkstoffs bei optimaler Verarbeitbarkeit der Komponenten der Arzneiform. Wie oben beschrieben sind zu hohe Anteile an Emulgator ebenso wie zu hohe Anteile an Retardierungsmittel an der Arzneiform nachteilig für die Verarbeitbarkeit und die Freisetzung des ersten und/oder zweiten Wirkstoffs. Es hat sich daher als besonders vorteilhaft erwiesen, eine Kombination von Emulgator und Retardierungsmittel zu verwenden, die wenigstens 40 Gew.-% der Gesamtmasse der Arzneiform ausmacht. Weiter bevorzugt beträgt die Summe der Gewichtsanteile an Emulgator und Retardierungsmittel an der erfindungsgemäßen Arzneiform wenigstens 45%, noch weiter bevorzugt wenigstens 48% und ganz besonders bevorzugt wenigstens 52%.

Dabei hat sich ein Masseverhältnis von Emulgator zu Retardierungsmittel von wenigstens 1:10, bevorzugt wenigstens 1:5 und noch mehr bevorzugt wenigstens 1:2 sowie besonders bevorzugt von wenigstens 1:1,2 als vorteilhaft erwiesen. Das Masseverhältnis beträgt vorzugsweise höchstens 10:1, weiter bevorzugt höchstens 5:1, noch weiter bevorzugt höchstens 2:1 und besonders bevorzugt höchstens 1,2:1. in ganz besonders bevorzugten Ausführungsformen der erfindungsgemäßen Arzneiform beträgt das Masseverhältnis von Emulgator zu Retardierungsmittel 1:1, in diesen Ausführungsformen sind Emulgator und Retardierungsmittel in einem Anteil von jeweils wenigstens 20 Gew.-% an der erfindungsgemäßen Arzneiform enthalten.

Das Masseverhältnis von zweitem Wirkstoff zu Retardierungsmittel beträgt erfindungsgemäß weniger als 1:1, weiter bevorzugt höchstens 1:1,2, noch weiter bevorzugt höchstens 1:1,5 und besonders bevorzugt höchstens 1:1,8. Bei zu geringen Anteilen an Retardierungsmittel im Verhältnis zum zweiten Wirkstoff wird die Freisetzung des zweiten Wirkstoffs nicht ausreichend modifiziert. Dann besteht die Gefahr, dass zu viel zweiter Wirkstoff bereits im Magen freigesetzt wird mit der Folge toxischer Plasmaspiegel. Allerdings sollte das Masseverhältnis einen Wert von 1:20, weiter bevorzugt 1:15, noch weiter bevorzugt von 1:10 und besonders bevorzugt von 1:4 nicht unterschreiten. Bei zu hohen Mengenanteilen von Retardierungsmittel wird die Freisetzung des zweiten Wirkstoffs zu stark verzögert und dieser möglicherweise erst in tiefen Darmabschnitten freigesetzt. Dann werden nur unzureichende Plasmaspiegel erreicht. Außerdem wird die Arzneiform insgesamt zu groß und damit schwerer schluckbar.

Die Arzneiform kann optional wenigstens ein Bindemittel umfassen. Das Bindemittel dient dazu, die anderen Komponenten der erfindungsgemäßen Arzneiform aneinanderzubinden, auch als "Bindekapazität" des Bindemittels bezeichnet. Das Bindemittel ist dabei insbesondere geeignet, den zweiten Wirkstoff an das Retardierungsmittel zu binden.

Die erfindungsgemäß eingesetzten Bindemittel haben den Vorteil, dass sie sich sehr leicht mit den anderen Komponenten der erfindungsgemäßen Arzneiformen verarbeiten lassen und schon in geringen Mengen ein hervorragendes Ergebnis liefern. Außerdem erleichtern die Bindemittel die Verarbeitung der Komponenten der erfindungsgemäßen Arzneiformen und tragen dazu bei, die Arzneiformen insgesamt zu stabilisieren, vorzugsweise die mechanische Stabilität der Arzneiformen bei der Herstellung zu erhöhen. Die Bindemittel sind beispielsweise geeignet, den Schmelzpunkt einer Mischung von erstem Wirkstoff und Emulgator während der Herstellung der erfindungsgemäßen Arzneiformen anzuheben, so dass eine weitere Bearbeitung dieser Mischung einfach möglich ist.

Geeignete Bindemittel können anorganischer oder organischer Art sein. Das Bindemittel ist vorzugsweise ein natürliches oder synthetisches Polysaccharid umfassend zwei oder mehrere gleiche oder verschiedene Monosaccharidbausteine, besonders bevorzugt ausgewählt aus Glucose, Galactose und Mischungen daraus. Solche Stoffe sind kostengünstig erhältlich, leicht verarbeitbar und zeigen teilweise gleichzeitig eine gewisse zerfallsfördernde Wirkung. Es ist besonders vorteilhaft, wenn das Bindemittel zugleich geeignet ist, einen schlechten Geschmack des ersten und/oder zweiten Wirkstoffs zu maskieren. Bevorzugte Bindemittel haben daher einen süßen Geschmack.

Besonders bevorzugt ist das Bindemittel ausgewählt aus Lactose, Stärke, Stärkederivaten und Mischungen daraus. Unter den Stärken ist Maisstärke besonders bevorzugt wegen der einfachen Verarbeitbarkeit und vorteilhaften Eigenschaften. Ganz besonders bevorzugt ist das Bindemittel ausgewählt aus Lactose, Maisstärke und Mischungen daraus, weiter bevorzugt besteht das Bindemittel aus Lactose, Maisstärke oder Mischungen daraus.

Der Anteil dieses Bindemittels an der Arzneiform soll vorzugsweise wenigstens 5 Gew.-%, weiter bevorzugt wenigstens 10 Gew.-% und besonders bevorzugt wenigstens 12 Gew.-% betragen. Vorzugsweise soll der Anteil an Bindemittel an der Arzneiform einen Wert von 40 Gew.-%, weiter bevorzugt 30 Gew.-% und besonders bevorzugt 20 Gew.-% nicht übersteigen. Eine zu große Menge an Bindemittel vergrößert die Arzneiform und ist daher unerwünscht.

In Ausführungsformen der Erfindung wird allerdings auf den Zusatz eines Bindemittels verzichtet. Die erfindungsgemäßen Arzneiformen sind dann noch schneller und kostengünstiger erhältlich bei sehr guter Stabilität.

Die erfindungsgemäßen Arzneiformen können Gleitmittel umfassen, insbesondere wenn ein Verpressen der Komponenten der erfindungsgemäßen Arzneiformen erwünscht ist.

Erfindungsgemäße Gleitmittel sind geeignet, die Herstellung der Arzneiformen weiter zu erleichtern, erfindungsgemäß als "Gleitwirkung" bezeichnet. in bevorzugten Ausführungsformen, in denen die erfindungsgemäßen Arzneiform als Tablette ausgestaltet ist, erleichtern die Gleitmittel beispielsweise das Verpressen der anderen Komponenten der Arzneiform zu einer Tablette.

Gleitmittel sind erfindungsgemäß ausgewählt aus Fließregulierungsmitteln, Schmiermitteln, Formentrennmitteln und Mischungen davon.

Vorzugsweise ist das Gleitmittel ausgewählt aus Stearinsäure, Stearaten, Calciumbehenat, hydrierten Pflanzenfetten, Talkum, hochdispersem Siliciumdioxid, Polyethylenglykolen, Natriumdodecylsulfat, Magnesiumdodecylsulfat und Mischungen daraus. Geeignete Stearate umfassen beispielsweise Magnesiumstearat, Glycerinmonostearat, Glyceropalmitostearat oder Mischungen daraus.

Bevorzugte Gleitmittel sind lipophil. Die Gleitmittel sind erfindungsgemäß bevorzugt praktisch unlöslich in wässriger Lösung bei 22°C und pH 7. Dies hat den Vorteil, dass die Gleitmittel insbesondere das Kleben der weiteren Komponenten der Arzneiform bei deren Verarbeitung an hierzu verwendeten Maschinen bzw. Apparaturen sowie die interpartikuläre Reibung optimal vermindern können. Weiter bevorzugt ist das Gleitmittel daher ausgewählt aus Stearinsäure, Stearaten, Calciumbehenat, hydrierten Pflanzenfetten, Talkum, hochdispersem Siliciumdioxid und Mischungen daraus.

Als besonders vorteilhaft hat es sich erwiesen, wenn das Gleitmittel ausgewählt ist aus Magnesiumstearat, Talkum, hochdispersem Siliciumdioxid und Mischungen daraus. Ganz besonders bevorzugt wird eine Mischung aus allen drei Komponenten als Gleitmittel verwendet, die Mischung wird auch als "Gleitmittelmischung" bezeichnet. Dies hat den Vorteil, dass die Eigenschaften aller drei Komponenten zum Vorteil der erfindungsgemäßen Arzneiformen eingesetzt werden können und sich die Wirkungen der Komponenten optimal ergänzen bzw. verstärken.

Allerdings muss dabei darauf geachtet werden, dass hochdisperses Siliciumdioxid in der Gleitmittelmischung nicht in zu hoher Menge enthalten ist. Zu hohe Mengen an hochdispersem Siliciumdioxid können die Gleitwirkung von Magnesiumstearat nachteilig beeinflussen und vermindern. Daher ist der Gehalt an hochdispersem Siliciumdioxid an der Gleitmittelmischung auf vorzugsweise 25 Gew.-%, weiter bevorzugt 20 Gew.-% und besonders bevorzugt 18 Gew.-% zu begrenzen.

Vorzugsweise sollte der Magnesiumstearatanteil im Verhältnis zum Anteil an Talkum in der Gleitmittelmischung gering sein. Das Masseverhältnis von Magnesiumstearat zu Talkum sollte vorzugsweise höchstens 1:1,3, weiter bevorzugt höchstens 1:1,5, noch weiter bevorzugt höchstens 1:1,8 betragen. War der Anteil an Magnesiumstearat höher im Verhältnis zum Talkanteil, so wurde eine Verschlechterung der Gleitwirkung von Magnesiumstearat bei gleichzeitiger Anwesenheit von Talkum beobachtet.

Eine bevorzugte Gleitmittelmischung umfasst:
- zwischen 5 Gew.-% und 25 Gew.-% hochdisperses Siliciumdioxid, weiter bevorzugt zwischen 8 Gew.-% und 17 Gew.-%; und
- zwischen 20 Gew.-% und 40 Gew.-% Magnesiumstearat, weiter bevorzugt zwischen 22 Gew.-% und 35 Gew.-%; und
- zwischen 45 Gew.-% und 75 Gew.-% Talkum, weiter bevorzugt zwischen 50 Gew.-% und 65 Gew.-%.

Der Anteil an Gleitmittel in den erfindungsgemäßen Arzneiformen ist vorzugsweise auf höchstens 15 Gew.-%, weiter bevorzugt höchstens 12 Gew.-% und mehr bevorzugt höchstens 10 Gew.-% begrenzt. Ein zu hoher Gehalt an Gleitmittel kann eine Entmischung bei der Herstellung der Arzneiform begünstigen, so dass die hervorragende Gleichförmigkeit des Gehalts der erfindungsgemäßen Arzneiform nicht mehr gewährleistet ist. Außerdem kann die Benetzbarkeit der erfindungsgemäßen Arzneiformen und damit deren Zerfall nachteilig beeinflusst werden.

in bevorzugten Ausführungsformen, in denen ein Gleitmittel in der erfindungsgemäßen Arzneiform enthalten ist, beträgt der Mindestgehalt an Gleitmittel vorzugsweise wenigstens 1 Gew.-%, weiter bevorzugt wenigstens 2 Gew.-% und besonders bevorzugt wenigstens 4 Gew.-% bezogen auf die Gesamtmasse der erfindungsgemäßen Arzneiform.

Die erfindungsgemäßen Arzneiformen können ferner optional sonstige Hilfsstoffe umfassen. Solche Hilfsstoffe können ausgewählt sein aus Füllmitteln, weiteren Bindemitteln, Tensiden, Fettalkoholen, Triglyceriden, Antioxidantien, Sprengmitteln, Komplexbildnern, Überzugsmitteln, Konservierungsmitteln, Weichmachern, Pigmenten und Mischungen aus solchen Substanzen. Der Fachmann ist in der Lage, entsprechend pharmazeutisch annehmbare Hilfsstoffe zu wählen. Dabei beträgt der Anteil optionaler sonstiger Hilfsstoffe an der erfindungsgemäßen Arzneiform vorzugsweise höchstens 10 Gew.-%, weiter bevorzugt höchstens 8 Gew.-% und besonders bevorzugt höchstens 5 Gew.-% bezogen auf die Gesamtmasse der Arzneiform.

in besonders bevorzugten Ausführungsformen der erfindungsgemäßen Arzneiform sind keine sonstigen Hilfsstoffe in der Arzneiform enthalten, die erfindungsgemäße Arzneiform besteht also aus erstem und zweiten Wirkstoff, Emulgator, Retardierungsmittel, optional Bindemittel und optional Gleitmittel.

Zusammenfassend kann die Arzneiform unter anderem enthalten:
a. einen ersten Wirkstoff (vorzugsweise in einem Anteil von wenigstens 1 Gew.-% und höchstens 25 Gew.-% bezogen auf die Gesamtmasse der Arzneiform),
b. einen zweiten Wirkstoff (vorzugsweise in einem Anteil von wenigstens 4 Gew.-% und höchstens 30 Gew.-% bezogen auf die Gesamtmasse der Arzneiform),
c. einen Emulgator zur Verbesserung der Auflösung des ersten Wirkstoffs und zur Modifikation der Freisetzung des ersten und des zweiten Wirkstoffs (vorzugsweise in einem Anteil von wenigstens 15 Gew.-% und höchstens 50 Gew.-% bezogen auf die Gesamtmasse der Arzneiform), insbesondere zur Verbesserung der Löslichkeit des ersten Wirkstoffes bei hohen pH-Werten und zur Verlangsamung der Auflösung des ersten Wirkstoffes bei niedrigen pH-Werten;
d. ein Retardierungsmittel zur Modifikation der Freisetzung des zweiten Wirkstoffes (vorzugsweise in einem Anteil von wenigstens 15 Gew.-% und höchstens 55 Gew.-% bezogen auf die Gesamtmasse der Arzneiform), insbesondere zur Verlangsamung der Auflösung des zweiten Wirkstoffs bei niedrigen pH-Werten;
e. optional ein Bindemittel zur Unterstützung bei der Verarbeitung der anderen Komponenten der erfindungsgemäßen Arzneiform während der Herstellung und zur Optimierung der mechanischen Stabilität (vorzugsweise in einem Anteil von wenigstens 5 Gew.-% und höchstens 40 Gew.-% bezogen auf die Gesamtmasse der Arzneiform);
f. optional ein Gleitmittel zur Verbesserung der Verarbeitbarkeit der anderen Komponenten der erfindungsgemäßen Arzneiform, insbesondere des Verpressens der anderen Komponenten (vorzugsweise in einem Anteil von wenigstens 1 Gew.-% und höchstens 15 Gew.-% bezogen auf die Gesamtmasse der Arzneiform);
g. optional sonstige Hilfsstoffe
h. optional weitere Wirkstoffe;
wobei die Summe der Gewichtsanteile von Emulgator und Retardierungsmittel wenigstens 40 Gew.-% bezogen auf die Arzneiform beträgt.

im Stand der Technik wird keine überzeugende Arzneiform vorgestellt, die es ermöglicht, sowohl einen gering löslichen Wirkstoff (hier: erster Wirkstoff) als auch einen zweiten Wirkstoff mit besserer Löslichkeit in einer Arzneiform jeweils modifiziert freizusetzen. Schließlich muss die Freisetzung des gering löslichen Wirkstoffes unterstützt werden und gleichzeitig die Freisetzung des besser löslichen Wirkstoffes unterdrückt werden. Diese Erfindung bietet eine Lösung für dieses Problem, indem eine erfindungsgemäße Arzneiform bereitgestellt wird, die beide Wirkstoffe umfasst zusammen mit wenigstens einem Emulgator und wenigsten einem Retardierungsmittel.

Im Kontext der hier beschriebenen Arzneiform war es eine Herausforderung, die Freisetzung des zweiten Wirkstoffes zu verzögern. Ein Grund ist, dass zur Lösungsvermittlung des ersten Wirkstoffes eine vergleichsweise große Menge an Emulgator benötigt wird. Dies führt dazu, dass das Volumen der Arzneiform bereits durch die notwendige löslichkeitsverbessernde Maßnahme hinsichtlich des ersten Wirkstoffes fast ausgereizt ist. Die freisetzungsverzögernde Maßnahme hinsichtlich des zweiten Wirkstoffes muss also in minimalem Volumen möglich sein. Dies ist dadurch möglich, dass ein Retardierungsmittel enthalten ist zur Modifikation der Freisetzung des zweiten Wirkstoffs und der Emulgator derart gewählt ist, dass dieser gleichzeitig auch zur Modifikation der Freisetzung des zweiten Wirkstoffs beiträgt. Dafür sind Anteile an Emulgator und Retardierungsmittel notwendig, die zusammen wenigstens 40 Gew.-% der Arzneiform ausmachen.

Ein Überzug über die ganze Arzneiform ist nicht bevorzugt, weil dieser Überzug die Freisetzung beider Wirkstoffe nachteilig beeinflussen und insbesondere nachteilig verzögern könnte. Es ist also erfindungsgemäß bevorzugt, dass die Arzneiform keinen Überzug aufweist, insbesondere keinen Überzug, der sich verzögert zeitabhängig und/oder der sich pH-abhängig löst. Die erfindungsgemäße Arzneiform weist also insbesondere keinen Überzug auf umfassend Cellulosederivate, Methacrylsäurepolymere, Polyvinylderivate, Schellack und Mischungen davon.

Eine besonders bevorzugte Ausführungsform der erfindungsgemäßen Arzneiform umfasst als ersten Wirkstoff Cinnarizin und als zweiten Wirkstoff Dimenhydrinat. Die Arzneiform ist zur Verwendung in der Therapie von Schwindel jedweder Genese geeignet. Daher ist auch die Verwendung dieser Arzneiform zur Behandlung von Schwindel jedweder Genese erfindungsgemäß. Erfindungsgemäß ist auch ein Verfahren zur Verabreichung der erfindungsgemäßen Arzneiform an einen Patienten, der einer Behandlung mit einem oder beiden der in der Arzneiform enthaltenen Wirkstoffe bedarf, insbesondere an einen Patienten, der an Schwindel gleich welcher Genese leidet.

Die Arzneiformen der Erfindung haben den Vorteil, dass sie besonders lagerstabil sind, dass heißt die Kriterien für Lagerstabilität der ICH sind mindestens erfüllt und vorzugsweise übertroffen, d.h. die erfindungsgemäßen Arzneiformen zeigen bessere Werte als ausreichend. Lagerstabilität bedeutet, dass während einer Lagerung bei bestimmten Lagerbedingungen und einer bestimmten Lagerdauer, die sich aus der ICH-Leitlinie Q3B (R2) (Impurities in New Drug Products) ergeben, ein ausreichend hoher Arzneistoffgehalt von mehr als 90%, bezogen auf die ursprüngliche Wirkstoffmenge, zur Verfügung steht, und dass Abbauprodukte, die eine Gefährdung der Patienten darstellen könnten, ein bestimmtes Höchstmaß nicht überschreiten. Die Höchstmaße ergeben sich aus der ICH-Leitlinie Q3B (R2). Die erfindungsgemäßen Arzneiformen erwiesen sich als äußerst lagerstabil in einer Kurzzeit-Stressuntersuchung bei 40°C und 75% relative Luftfeuchte gemäß ICH-Leitlinie Q3B (R2) über 6 Monate. Die erfindungsgemäßen Arzneiformen enthielten nach der Lagerung noch mehr als 90% des Wirkstoffs bezogen auf die Menge an Wirkstoff in der nicht gelagerten Arzneiform und die Menge an Verunreinigungen lag innerhalb der zulässigen Grenzen gemäß ICH-Leitlinie Q3B (R2). Die erfindungsgemäßen Arzneiformen sind vorzugsweise lagerstabil über mehr als 6 Monate, bevorzugt wenigstens 12 Monate Lagerdauer unter Standardbedingungen gemäß internationaler Leitlinien der ICH.

Die erfindungsgemäßen Arzneiformen zeichnen sich durch eine exzellente Gleichförmigkeit der Masse und Gleichförmigkeit des Gehalts aus, was durch die Zusammensetzung der Arzneiformen und das Herstellungsverfahren gewährleistet ist. Die Prüfung erfolgt nach den entsprechenden Methoden des Europäischen Arzneibuchs (Ph.Eur. 7). Eine Arzneiform zeigt vorzugsweise eine Gleichförmigkeit der Masse derart, dass die Masse von 20 solcher Arzneiformen vorzugsweise um weniger als 5%, weiter bevorzugt um weniger als 4% von der Durchschnittsmasse der Arzneiform abweicht, die sich aus der Masse der 20 Arzneiformen ergibt. Die erfindungsgemäße Arzneiform zeigt bevorzugt eine Gleichförmigkeit des Gehalts derart, dass der Wirkstoffgehalt von 10 solcher Arzneiformen jeweils zwischen 85% und 115%, bevorzugt zwischen 87% und 113% und idealerweise zwischen 90% und 110%, bezogen auf den Durchschnittsgehalt der 10 Arzneiformen an Wirkstoff liegt.

Bevorzugt ist die erfindungsgemäße Arzneiform als Tablette ausgestaltet, diese weist weiter bevorzugt eine hervorragende mechanische Stabilität auf. Als Maß für die mechanische Stabilität der erfindungsgemäßen Arzneiformen kann die Bruchfestigkeit und die Friabilität herangezogen werden.

Die Tablette weist bevorzugt eine Bruchfestigkeit von mindestens 40 N, weiter bevorzugt mindestens 50 N auf. Die Bruchfestigkeit sollte vorzugsweise aber 200 N, bevorzugt 150 N, weiter bevorzugt 130 N und besonders bevorzugt 100 N nicht übersteigen. Bei zu hohen Bruchfestigkeiten ist der Zerfall der Tablette messbar schlechter. Die Bruchfestigkeit einer Tablette wird nach der entsprechenden Standardmethode des Europäischen Arzneibuchs bestimmt mit einem Gerät bestehend aus zwei Backen (Ph.Eur. 7), wobei die Prüfung an 10 Tabletten durchgeführt wird.

Vorzugsweise beträgt der Masseverlust der erfindungsgemäßen Arzneiformen bei der Friabilitätsprüfung nach Europäischem Arzneibuch (Ph.Eur. 7) von wenigstens 10 Arzneiformen weniger als 1%, vorzugsweise sogar weniger als 0,8% und mehr bevorzugt weniger als 0,6%. Die Anforderungen an die Friabilität des Europäischen Arzneibuchs werden also vorzugsweise übertroffen.

Die erfindungsgemäßen Arzneiformen haben ferner den Vorteil, dass sie flexibel eingesetzt werden können. So ist es möglich, an die erfindungsgemäße Arzneiform vorliegend als Tablette wenigstens eine zusätzliche wirkstoffhaltige Schicht anzubringen, erfindungsgemäß als "Zusatzschicht" bezeichnet.

in Ausführungsformen der vorliegenden Erfindung liegt die erfindungsgemäße Arzneiform daher als Tablette vor, an die wenigstens eine Zusatzschicht angebracht sein kann, vorzugsweise genau eine Zusatzschicht. Die Zusatzschicht kann wenigstens einen Wirkstoff modifiziert oder sofort freisetzen. "Sofortige Freisetzung" bedeutet dabei vorzugsweise, dass mindestens 65% des Wirkstoffs, bevorzugt mindestens 75% und weiter bevorzugt mindestens 80% des Wirkstoffs nach 60 Minuten in das Prüfmedium, bevorzugt Magensaft, freigesetzt werden. Die Messung kann nach üblichen Methoden der Freisetzungsmessung erfolgen, insbesondere der Methoden nach Europäischen Arzneibuch oder des Amerikanischen Arzneibuchs (USP), bevorzugt mit der Bio-Dis-Apparatur (200 ml Prüfmedium, 25 dpM, 37°C). Die konkreten Bedingungen hängen vom jeweiligen Wirkstoff und der Arzneiform ab und sind gängigen Arzneibüchern zu entnehmen.

in den Ausführungsformen, in denen an die Arzneiform eine Zusatzschicht angebracht ist, ist es bevorzugt, dass die Zusatzschicht nur einen Teil der Arzneiform bedeckt, um die Freisetzung des ersten und zweiten Wirkstoffs aus der Arzneiform nicht zu behindern. Außerdem kann die Gefahr bestehen, dass die Schluckbarkeit verschlechtert ist, wenn die Zusatzschicht die Arzneiform vollständig umschließt, also die Gesamtoberfläche der Arzneiform vollständig von der Zusatzschicht bedeckt ist. Es ist vorteilhaft, wenn die Zusatzschicht maximal 70%, weiter bevorzugt maximal 65% und besonders bevorzugt maximal 50% der Gesamtoberfläche der Arzneiform bedeckt.

Die Zusatzschicht sollte zudem keine zu hohe Masse aufweisen, damit die Arzneiform und die Zusatzschicht zusammen noch gut schluckbar sind. Daher beträgt die Masse der Zusatzschicht maximal 400 mg, bevorzugt maximal 350 mg und besonders bevorzugt maximal 300 mg. Allerdings ist eine gewisse Mindestmasse erforderlich, damit die Zusatzschicht an die Arzneiform angebracht werden kann. Daher beträgt die Masse der Zusatzschicht vorzugsweise wenigstens 100 mg, weiter bevorzugt wenigstens 150 mg.

Vorteilhaft ist es, wenn das Masseverhältnis von Zusatzschicht zu Arzneiform maximal 1:1,5, bevorzugt maximal 1:1,8 und weiter bevorzugt maximal 1:2 beträgt. Bei solchen Verhältnissen war eine gute Schluckbarkeit gegeben.

Die Zusatzschicht kann den ersten und/oder den zweiten Wirkstoff umfassen. Vorteilhaft ist es, wenn die Zusatzschicht den ersten und den zweiten Wirkstoff enthält und beide Wirkstoffe sofort freisetzt. Dies ergänzt die modifizierte Freisetzung des ersten und zweiten Wirkstoffs aus der Arzneiform.

Es kann beispielsweise bei Langzeittherapien mit bestimmten Wirkstoffen vorteilhaft sein, schnell einen therapeutischen Plasmaspiegel zu erzielen, insbesondere wenn bei langen Dosierungsintervallen gegen Ende des Intervalls die minimale Wirkkonzentration unterschritten wird. Auch bei Kurzeittherapien kann ein schnelles Erreichen therapeutischer Plasmaspiegel erwünscht sein. Durch die erfindungsgemäße Arzneiform, die den ersten und zweiten Wirkstoff modifiziert freisetzt, wird die Wirkung durch die nachgelagerte Freisetzung anschließend in die Länge gezogen.

Sind erster und zweiter Wirkstoff in der Zusatzschicht enthalten, so hat sich ein Masseverhältnis von erstem Wirkstoff in der Arzneiform zu erstem Wirkstoff in der Zusatzschicht bzw. ein Masseverhältnis von zweitem Wirkstoff in der Arzneiform zu zweitem Wirkstoff in der Zusatzschicht zwischen 1:1 und 2,5:1 als vorteilhaft erwiesen. Eine solche Wirkstoffverteilung trägt dazu bei, einen optimalen Verlauf der Plasmaspiegel insbesondere bei sofortiger Freisetzung aus der Zusatzschicht zu erzielen ohne toxische Plasmaspiegel und unerwünschte Plasmaspiegelschwankungen.

Wenn der Wirkstoffanteil in der Zusatzschicht zu hoch ist, kann es sein, dass insbesondere bei sofortiger Freisetzung aus der Zusatzschicht zu hohe Plasmaspiegel an erstem und/oder zweitem Wirkstoff bzw. ein zu steiler Anstieg der Plasmaspiegel resultieren. Die Menge an erstem bzw. zweitem Wirkstoff in der Zusatzschicht ist bevorzugt höchstens so hoch, wie die entsprechende Menge an erstem bzw. zweitem Wirkstoff in der Arzneiform.

Zum anderen ist es sinnvoll, den Gehalt an erstem bzw. zweitem Wirkstoff in der Zusatzschicht im Verhältnis zum Anteil an erstem bzw. zweitem Wirkstoff in der Arzneiform ausreichend hoch zu wählen. Es hat sich als erfindungsgemäß vorteilhaft erwiesen, wenn das Masseverhältnis von erstem Wirkstoff in der Arzneiform zu erstem Wirkstoff in der Zusatzschicht bzw. das Masseverhältnis von zweitem Wirkstoff in der Arzneiform zu zweitem Wirkstoff in der Zusatzschicht einen Wert von 2,5:1 nicht überschreitet, bevorzugt einen Wert von 2,2:1 und weiter bevorzugt einen Wert von 2,1:1. ist der Wirkstoffgehalt in der Zusatzschicht zu gering, besteht die Gefahr, dass keine ausreichenden Plasmaspiegel an erstem und zweitem Wirkstoff erzielt werden, insbesondere bei sofortiger Freisetzung des ersten und zweiten Wirkstoffs aus der Zusatzschicht. Außerdem kann bei einem ausreichend hohen Gehalt an erstem und zweitem Wirkstoff in der Zusatzschicht die physiologische Retardierung aufgrund der Verweilzeit der Arzneiform im Magen vorteilhaft ausgenutzt werden. Durch die Verweilzeit im Magen unterliegt eine zusätzlich vorhandene Zusatzschicht nämlich einer gewissen physiologischen Retardierung. Bei ausreichend hohem Gehalt an erstem und zweitem Wirkstoff in der Zusatzschicht ist es beispielsweise möglich, den Wirkstoffgehalt in der erfindungsgemäßen Arzneiform insgesamt zu verringern. Dadurch kann die Größe der Arzneiform zusätzlich verringert werden, was sich positiv auf deren Schluckbarkeit und die Patienten-Compliance auswirken kann.

in einer erfindungsgemäßen Ausführungsform liegt das Masseverhältnis von erstem Wirkstoff in der Arzneiform zu erstem Wirkstoff in der Zusatzschicht bzw. das Masseverhältnis von zweitem Wirkstoff in der Arzneiform zu zweitem Wirkstoff in der Zusatzschicht bei 2:1.

Außerdem kann die Zusatzschicht Hilfsstoffe enthalten beispielsweise ausgewählt aus Füllstoffen, Presshilfsmitteln, Bindemitteln, Tensiden, Fettalkoholen, Triglyceriden, Antioxidantien, Sprengmitteln, Komplexbildnern, Überzugsmitteln, Konservierungsmitteln, Weichmachern, Verzögerungsmitteln, Pigmenten und Mischungen aus solchen Substanzen. Der Fachmann ist in der Lage, entsprechend pharmazeutisch annehmbare Hilfsstoffe sowie geeignete Mengenanteile zu wählen.

Wenn die Zusatzschicht den ersten und zweiten Wirkstoff sofort freisetzen soll, haben sich Anteile an Hilfsstoffen ausgewählt aus Füllstoffen, Sprengmitteln und Presshilfsmitteln als geeignet erwiesen. Der Anteil der Hilfsstoffe an der Zusatzschicht kann bis zu 95 Gew.-%, weiter bevorzugt bis zu 90 Gew.-% und noch weiter bevorzugt bis zu 85 Gew.-% betragen. Zu hohe Anteile an Hilfsstoffen führen dazu, dass die Zusatzschicht insgesamt zu groß wird.

Geeignete Füllstoffe für eine Zusatzschicht sind solche, die gleichzeitig eine gute Verpressbarkeit zeigen. So darf die Arzneiform beim Aufpressen der Zusatzschicht nicht beschädigt werden. Als vorteilhaft haben sich Füllstoffe ausgewählt aus mikrokristalliner Cellulose, Calciumhydrogenphosphat-Dihydrat und Mischungen davon erwiesen, wobei ein Anteil zwischen 40 Gew.-% und 65 Gew.-% an Füllstoff bezogen auf die Gesamtmasse der Zusatzschicht günstig ist.

Wenn die Zusatzschicht den ersten und zweiten Wirkstoff sofort freisetzen soll, ist es vorteilhaft, wenn ein Sprengmittel in der Zusatzschicht enthalten ist. Vorteilhaft sind Anteile zwischen 10 Gew.-% und 30 Gew.-%. Geeignete Sprengmittel sind Stärke, Stärkederivate oder Mischungen daraus. Vorteilhaft ist beispielsweise vorverkleisterte Stärke wie Starch 1500.

Presshilfsmittel erleichtern die Herstellung und Verarbeitung der Zusatzschicht und können insbesondere Gleitmittel umfassen wie hochdisperses Siliciumdioxid, Magnesiumstearat oder Mischungen daraus. Vorteilhafte Anteile von Presshilfsmittel an der Zusatzschicht liegen zwischen 0,25 Gew.-% und 5 Gew.-%.

Eine Zusatzschicht kann also enthalten:
a. einen oder mehrere Wirkstoffe, insbesondere den ersten und den zweiten Wirkstoff;
b. Hilfsstoffe, beispielsweise ausgewählt aus Füllstoffen, Sprengmitteln, Presshilfsmitteln und Mischungen daraus.

Die Erfindung betrifft auch ein Herstellungsverfahren für die erfindungsgemäße Arzneiform. Ein großer Vorteil der erfindungsgemäßen Arzneiformen ist, dass sie kostengünstig und schnell herstellbar sind, das Herstellungsverfahren also nur wenige und gering aufwändige Verfahrensschritte erfordert.

Das erfindungsgemäße Herstellungsverfahren umfasst die Schritte:
a. Mischen von Komponenten der Arzneiform;
b. Granulieren der Komponenten; und
c. Herstellung einer monolithischen Arzneiform aus dem Granulat.

Es werden zunächst Komponenten der erfindungsgemäßen Arzneiform gemischt, vorzugsweise wenigstens der erste Wirkstoffs, der zweite Wirkstoff, der Emulgator, das Retardierungsmittel und optional das Bindemittel.

Das Mischen der Komponenten umfasst bevorzugt das Herstellen einer sogenannten "Mischung I" und einer "Mischung II".

Mischung I umfasst wenigstens den erstem Wirkstoff und den Emulgator sowie optional andere Komponenten. Optional kann auch ein Lösungsmittel enthalten sein. Je nach genauer Beschaffenheit von Wirkstoff und Emulgator sind verschiedene Lösungsmittel denkbar. Vorzugsweise werden in der pharmazeutischen Technologie übliche Lösungsmittel verwendet, insbesondere Alkohole, Ester und/oder Ketone, insbesondere Aceton. Besonders bevorzugt besteht Mischung I aus dem ersten Wirkstoff und dem Emulgator.

Mischung I kann hergestellt werden durch Vermengen wenigstens von erstem Wirkstoff und Emulgator, wobei dem Fachmann bekannte Mischer verwendet werden können wie beispielsweise ein Zwangsmischer. Bevorzugt erfolgt das Vermengen dadurch, dass der Emulgator geschmolzen wird und der erste Wirkstoff und optional weitere Komponenten in der Schmelze gelöst werden. in einer alternativen Ausführungsform werden erster Wirkstoff und Emulgator und optional weitere Komponenten gemischt, insbesondere in einem Zwangsmischer, und anschließend geschmolzen.

Mischung II umfasst den zweiten Wirkstoff, das Retardierungsmittel und optional weitere Komponenten, insbesondere das Bindemittel. Besonders bevorzugt besteht Mischung II aus dem zweiten Wirkstoff, dem optionalen Bindemittel und dem Retardierungsmittel. Mischung II wird hergestellt durch Vermengen wenigstens von zweitem Wirkstoff, Retardierungsmittel und optional Bindemittel. Dies kann mit dem Fachmann bekannten Mischern erfolgen, beispielsweise einem Zwangsmischer.

Die Komponenten der erfindungsgemäßen Arzneiform werden dann granuliert zum Erhalt eines Granulats. Dies kann dadurch erfolgen, dass Mischung I und Mischung II mit dem Fachmann bekannten Granulierverfahren granuliert werden, insbesondere ausgewählt aus Mischgranulierung, Schmelzgranulierung oder Sprühgranulierung beispielsweise als Wirbelschichtgranulierung.

in erfindungsgemäß bevorzugten Ausführungsformen wird ein Granulat mit Hilfe eines Sprühverfahrens hergestellt, bevorzugt durch Aufsprühen von Mischung I auf Mischung II, gegebenenfalls unter Verwendung eines Lösungsmittels. Bevorzugt erfolgt das Aufsprühen in einer Wirbelschichtanlage. Weiter bevorzugt erfolgt das Aufsprühen ohne Verwendung eines Lösungsmittels. Es hat sich als besonders vorteilhaft erwiesen, Mischung II in einer Wirbelschichtanlage vorzulegen und Mischung I anschließend auf die vorgelegte Mischung II aufzusprühen. Ein solches Verfahren ermöglicht ein schnelles und kosteneffektives Granulieren. Die erhaltenen Granulate weisen für die Weiterverarbeitung optimal geeignete Größen auf und sehr gute Fließeigenschaften, so dass auch die Herstellung der monolithischen Arzneiform aus dem Granulat schnell und kosteneffektiv erfolgen kann.

Alternativ kann das Granulieren dadurch erfolgen, dass Mischung I auf Mischung II "aufgranuliert" wird. Das Aufgranulieren erfolgt erfindungsgemäß durch einfache Mischgranulierung in einem gängigen Mischer oder Mischkneter. Auch mit einem solchen Granulieren kann ein für die Herstellung der erfindungsgemäßen Arzneiform geeignetes Granulat erhalten werden. Das Aufgranulieren ist erfindungsgemäß besonders vorteilhaft, wenn Granulate mit besonders gutem Zusammenhalt und guter Plastizität gewünscht sind.

in alternativen Ausführungsformen erfolgt das Granulieren von Mischung I und Mischung II durch Vermengen von Mischung I und Mischung II unter Herstellung eines Schmelzgranulats.

Dann wird aus dem Granulat eine monolithische Arzneiform hergestellt. Hierzu wird das Granulat vorzugsweise gesiebt, bevorzugt mit einer Maschenweite von 1 mm, um gleichmäßige Granulatkörner sicherzustellen. Dies ist auch vorteilhaft für eine gleichmäßige Freisetzung einerseits und andererseits eine gute Verarbeitbarkeit des Granulats.

Das bevorzugt gesiebte Granulat wird mit dem optionalen Gleitmittel und optional sonstigen Hilfsstoffen zu einer erfindungsgemäßen Arzneiform verarbeitet. im Falle einer Tablette wird das Granulat in einer Tablettenpresse zusammen mit dem optionalen Gleitmittel und optional sonstigen Hilfsstoffen verpresst. Dabei wird vorzugsweise eine Presskraft von höchstens 35 kN, weiter bevorzugt höchstens 30 kN und besonders bevorzugt höchstens 28 kN und ganz besonders bevorzugt höchstens 18 kN angelegt. Werden zu hohe Presskräfte eingesetzt, so werden häufig zu feste Arzneiformen erhalten mit einer damit verbundenen schlechteren Freisetzung der Wirkstoffe aus der Arzneiform. Die Presskraft sollte aber vorzugsweise 7,5 kN, weiter bevorzugt 8 kN übersteigen. Werden zu niedrige Presskräfte eingesetzt, ist eine unzureichende Festigkeit der Arzneiform zu beobachten.

Anschließend kann an die Arzneiform optional eine Zusatzschicht angebracht werden, vorzugsweise durch Aufpressen einer Zusatzschichtmischung, umfassend die Komponenten der Zusatzschicht. Die Zusatzschichtmischung kann auch als Granulat vorliegen herstellbar durch Granulieren der Komponenten der Zusatzschicht mit dem Fachmann bekannten Granulierverfahren.

Sofern erforderlich, können der erste und/oder zweite Wirkstoff vor Herstellung der erfindungsgemäßen Arzneiform zerkleinert werden, insbesondere in einer Nanomühle. Das so erhaltene Pulver des ersten Wirkstoffs wird vorzugsweise in den Emulgator eingelagert. Zur Einlagerung wird ein Lösungsmittel verwendet, in dem sich der erste Wirkstoff nicht löst, insbesondere Wasser. Die Einlagerung verhindert die Reagglomeration der Wirkstoffpartikel.

### Abbildungen

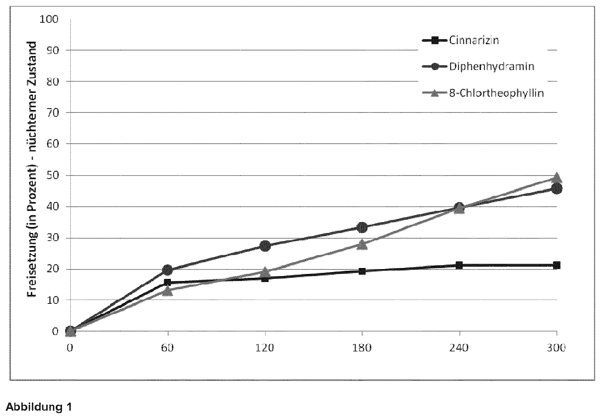

Abbildung 1 zeigt die Freisetzung eines ersten Wirkstoffs (Cinnarizin) und eines zweiten Wirkstoffs (Dimenhydrinat) aus einer erfindungsgemäßen Arzneiform nach Beispiel 1 im nüchternen Zustand. Die Messung erfolgte in 200 ml Prüfmedium mittels der Bio-Dis-Apparatur (25 dpM - dips pro Minute, 37°C). Die Messung der Wirkstoffmengen erfolgte mittels HPLC. Es wurde ein getaktetes Freisetzungsprofil gewählt, um den nüchternen Zustand möglichst physiologisch abzubilden. Dabei wurden als Prüfmedien in den ersten 60 Minuten 0,1 N HCl, nachfolgend für 120 Minuten FaSSIF-Puffer nach Dressman et al., anschließend für 60 Minuten 50% FaSSIF und anschließend für weitere 60 Minuten Puffer pH 7,0 gewählt. Die Ordinate zeigt die freigesetzte Wirkstoffmenge in Prozent, die Abszisse die vergangenen Minuten.

innerhalb von 60 Minuten werden weniger als 20% an Cinnarizin und Diphenhydramin bzw. 8-Chlortheophylin aus der Arzneiform bezogen auf die Menge an Wirkstoff in der Arzneiform freigesetzt. Nach 120 Minuten sind weniger als 30% aus der Arzneiform freigesetzt. Die Freisetzung dauert auch nach 5 Stunden noch an. Die Arzneiform setzt den ersten und zweiten Wirkstoff also optimal verzögert und verlängert frei.

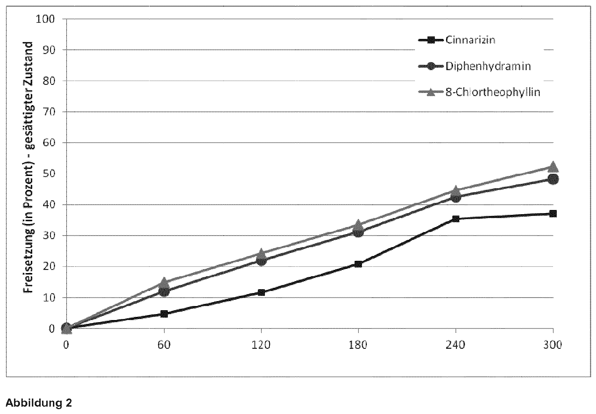

Abbildung 2 zeigt die Freisetzung eines ersten Wirkstoffs (Cinnarizin) und eines zweiten Wirkstoffs (Dimenhydrinat) aus einer erfindungsgemäßen Arzneiform nach Beispiel 1 im gesättigten Zustand. Die Messung erfolgte in 200 ml Prüfmedium mittels der Bio-Dis-Apparatur (25 dpM - dips pro Minute, 37°C). Die Messung der Wirkstoffmengen erfolgte mittels HPLC. Es wurde ein getaktetes Freisetzungsprofil gewählt, um den gesättigten Zustand möglichst physiologisch abzubilden. Dabei wurden als Prüfmedien in den ersten 60 Minuten FeSSGF nach Dressman et al., nachfolgend für 120 Minuten Pufferlösung pH 3,0 R, anschließend für 60 Minuten 0,01 N HCl und anschließend für weitere 60 Minuten FaSSIF nach Dressman et al. gewählt. Die Ordinate zeigt die freigesetzte Wirkstoffmenge in Prozent, die Abszisse die vergangenen Minuten.

innerhalb von 60 Minuten werden weniger als 20% an Cinnarizin und Diphenhydramin bzw. 8-Chlortheophylin aus der Arzneiform freigesetzt. Nach 120 Minuten sind weniger als 30% aus der Arzneiform freigesetzt. Die Freisetzung dauert auch nach 5 Stunden noch an. Die Arzneiform setzt den ersten und zweiten Wirkstoff also optimal verzögert und verlängert frei, selbst im gesättigten Zustand.

### Beispiele

### Beispiel 1

Es wurde eine Arzneiform der folgenden Zusammensetzung hergestellt:

| **Bestandteil** | **Menge (mg)** | **Funktion** |
|---|---|---|
| Cinnarizin | 60 | erster Wirkstoff |
| Dimenhydrinat | 120 | zweiter Wirkstoff |
| Methocel^{®} E4M (Hydroxypropylmethylcellulose) | 240 | Retardierungsmittel |
| Gelucire^{®} 50/13 | 240 | Emulgator |
| Lactose, wasserfrei | 120 | Bindemittel |
| **Gesamtmasse:** | **780** | |

Es wurde eine erfindungsgemäße Arzneiform mit dem Wirkstoff Cinnarizin (erster Wirkstoff) und dem weiteren Wirkstoff Dimenhydrinat (zweiter Wirkstoff) hergestellt. Cinnarizin ist eine schwache Base, die bei einem pH-Wert von 1 eine Wasserlöslichkeit von etwa 1,55 mg/ml aufweist. Die Löslichkeit über pH 7 liegt unter 0,01 mg/ml. Bei pH 7 beträgt die Löslichkeit beispielsweise unter etwa 0,00025 mg/ml. Damit ist Cinnarizin ein Wirkstoff gemäß der vorliegenden Erfindung mit geringer Löslichkeit, der zudem im sauren pH-Bereich des Magens protoniert vorliegt und im pH-Bereich des Darms praktisch unlöslich ist. Cinnarizin umfasst ferner zwei Aminogruppen. Cinnarizin ist also ein erster Wirkstoff gemäß der vorliegenden Erfindung. Zusätzlich ist Cinnarizin nur begrenzt lagerstabil und geschützt vor Licht zu lagern, was eine zusätzliche Herausforderung bei der Formulierung von Cinnarizin darstellt.

Dimenhydrinat weist eine höhere Löslichkeit auf als Cinnarizin bei pH 7. Die Löslichkeit ist um wenigstens eine Zehnerpotenz höher als die Löslichkeit von Cinnarizin bei pH 7. Dimenhydrinat ist also ein zweiter Wirkstoff gemäß der vorliegenden Erfindung. Auch weist Dimenhydrinat permanente Ladungen auf. Zusätzlich weist Dimenhydrinat einen bitteren und betäubenden Geschmack auf, was eine zusätzliche Herausforderung bei der Formulierung von Dimenhydrinat darstellt.

Zur Herstellung der Arzneiform wurden die angegebenen Komponenten nach dem erfindungsgemäßen Herstellungsverfahren verarbeitet. Zunächst wurden die Komponenten vermischt, wobei eine Mischung I und eine Mischung II hergestellt wurde. Die Mischung I wurde hergestellt durch Schmelzen von Emulgator (Gelucire^{®} 50/13) und Lösen von Cinnarizin als erster Wirkstoff in der Schmelze. Mischung II wurde erhalten durch Vermischen von Dimenhydrinat als zweiter Wirkstoff mit Retardierungsmittel (Methocel^{®} E4M) und Bindemittel (Lactose, wasserfrei). Anschließend wurden die Komponenten granuliert, indem Mischung I auf Mischung II aufgranuliert wurde. Das erhaltene Granulat wurde anschließend zu einer Tablette verpresst.

Die Abbildungen 1 und 2 zeigen die Freisetzung des ersten und zweiten Wirkstoffs aus der Arzneiform, wobei ein getaktetes Freisetzungsprofil gewählt wurde, um die physiologischen Zustände und Vorgänge möglichst genau abzubilden. Dabei wurde ein nüchterner Zustand (Abbildung 1) und ein gesättigter Zustand (Abbildung 2) nachgeahmt. Es ist erkennbar, dass die Freisetzung sowohl im nüchternen als auch im gesättigten Zustand langsam ansteigt, so dass eine verlängerte Wirkung erzielt werden kann. Die Gefahr eines Dose Dumping ist auch im gesättigten Zustand nicht gegeben. Selbst bei einer Verweilzeit der Arzneiform für vier Stunden im Magen im gesättigten Zustand, werden unter 36% Cinnarizin, unter 45% Diphenhydramin und unter 45% 8-Chlortheophylin aus der erfindungsgemäßen Arzneiform freigesetzt. Damit ist ein solcher Plasmaspiegelanstieg sowohl bei Einnahme der Arzneiform im nüchternen wie auch gesättigten Zustand zu erwarten, der die Reduktion der täglichen Einnahme auf 2 Arzneiformen, vorzugsweise 1 Arzneiform pro Tag zulässt, was zu einer Verbesserung der Patientencompliance beiträgt.

### Beispiel 2

Es wurde eine Arzneiform der folgenden Zusammensetzung hergestellt:

| **Bestandteil** | **Menge (mg)** | **Funktion** |
|---|---|---|
| Cinnarizin | 60 | erster Wirkstoff |
| Dimenhydrinat | 120 | zweiter Wirkstoff |
| Methocel^{®} E4M (Hydroxypropylmethylcellulose) | 240 | Retardierungsmittel |
| Gelucire^{®} 50/13 | 240 | Emulgator |
| Lactose, wasserfrei | 120 | Bindemittel |
| **Gesamtmasse:** | **780** | |

Zur Herstellung der Arzneiform wurden die angegebenen Komponenten nach dem erfindungsgemäßen Herstellungsverfahren verarbeitet. Zunächst wurden die Komponenten vermischt, wobei eine Mischung I und eine Mischung II hergestellt wurde. Die Mischung I wurde hergestellt durch Schmelzen von Emulgator (Gelucire^{®} 50/13) und Lösen von Cinnarizin als erster Wirkstoff in der Schmelze. Mischung II wurde erhalten durch Vermischen von Dimenhydrinat als zweiter Wirkstoff mit Retardierungsmittel (Methocel^{®} E4M) und Bindemittel (Lactose, wasserfrei).

Anschließend wurden die Komponenten granuliert, indem Mischung I auf die vorgelegte Mischung II aufgesprüht wurde in einer Wirbelschichtanlage. Das erhaltene Granulat zeigte für die Weiterverarbeitung optimale Eigenschaften, insbesondere hinsichtlich Fließeigenschaften und Größe der Granulatkörner und konnte anschließend leicht und schnell zu einer Tablette verpresst werden.

### Beispiel 3

Es wurde eine Arzneiform hergestellt, die folgende Zusammensetzung aufwies:

| **Bestandteil** | **Menge (mg)** | **Funktion** |
|---|---|---|
| Cinnarizin | 60 | erster Wirkstoff |
| Dimenhydrinat | 120 | zweiter Wirkstoff |
| Methocel^{®} E4M (Hydroxypropylmethylcellulose) | 240 | Retardierungsmittel |
| Gelucire^{®} 50/13 | 240 | Emulgator |
| Maisstärke | 120 | Bindemittel |
| Aerosil^{®} (hochdisperses Siliciumdioxid) | 7,8 | Gleitmittel |
| Magnesiumstearat | 15,6 | Gleitmittel |
| Talkum | 31,2 | Gleitmittel |
| **Gesamtmasse:** | **834,6** | |

Dabei wurden die angegebenen Komponenten nach dem erfindungsgemäßen Herstellungsverfahren verarbeitet. Zunächst wurden Komponenten vermischt, wobei eine Mischung I und eine Mischung II hergestellt wurde.

Die Mischung I wurde hergestellt durch Schmelzen von Emulgator (Gelucire^{®} 50/13) und Lösen von Cinnarizin als erster Wirkstoff in der Schmelze. Mischung II wurde erhalten durch Vermischen von Dimenhydrinat als zweiter Wirkstoff mit Retardierungsmittel (Methocel^{®} E4M) und Bindemittel (Maisstärke). Anschließend wurden die Komponenten granuliert, indem Mischung I auf Mischung II aufgranuliert wurde. Das erhaltene Granulat wurde anschließend zusammen mit der Gleitmittelmischung zu einer Tablette verpresst.

### Beispiel 4

Es wurde eine Arzneiform hergestellt, die folgende Zusammensetzung aufwies:

| **Bestandteil** | **Menge (mg)** | **Funktion** |
|---|---|---|
| Cinnarizin | 40 | erster Wirkstoff |
| Dimenhydrinat | 80 | zweiter Wirkstoff |
| Methocel^{®} E4M (Hydroxypropylmethylcellulose) | 160 | Retardierungsmittel |
| Gelucire^{®} 50/13 | 160 | Emulgator |
| Pharmatose^{®} (wasserfreie Lactose) | 80 | Bindemittel |
| Aerosil^{®} (hochdisperses Siliciumdioxid) | 5,2 | Gleitmittel |
| Magnesiumstearat | 10,4 | Gleitmittel |
| Talkum | 20,8 | Gleitmittel |
| **Gesamtmasse:** | **556,4** | |

Dabei wurden die angegebenen Komponenten nach dem erfindungsgemäßen Herstellungsverfahren verarbeitet. Zunächst wurden Komponenten vermischt, wobei eine Mischung I und eine Mischung II hergestellt wurde.

Die Mischung I wurde hergestellt durch Schmelzen von Emulgator (Gelucire^{®} 50/13) und Lösen von Cinnarizin als erster Wirkstoff in der Schmelze. Mischung II wurde erhalten durch Vermischen von Dimenhydrinat als zweiter Wirkstoff mit Retardierungsmittel (Methocel^{®} E4M) und Bindemittel (Pharmatose^{®}). Anschließend wurden die Komponenten granuliert, indem Mischung I auf Mischung II aufgranuliert wurde. Das erhaltene Granulat wurde anschließend zusammen mit der Gleitmittelmischung zu einer Tablette verpresst.

Beim Verpressen zur Herstellung der erfindungsgemäßen Arzneiform wurde an die Arzneiform eine Zusatzschicht angebracht mit folgender Zusammensetzung:

| **Bestandteil** | **Menge (mg)** | **Funktion** |
|---|---|---|
| Cinnarizin | 20 | erster Wirkstoff |
| Dimenhydrinat | 40 | zweiter Wirkstoff |
| Starch 1500 (vorverkleisterte Stärke) | 50 | Sprengmittel |
| Vivapur^{®} 101 (mikrokristalline Cellulose) | 80 | Füllstoff |
| Aerosil^{®} 200 (hochdisperses Siliciumdioxid) | 1,25 | Presshilfsmittel |
| Magnesiumstearat | 2,0 | Presshilfsmittel |
| Emcompress^{®} (Calciumhydrogenphosphat-Dihydrat) | 56,75 | Füllstoff |
| **Gesamtmasse:** | **250** | |

Zur Herstellung der Zusatzschicht wurden die Komponenten miteinander vermischt und auf die Arzneiform aufgepresst.

## Patentansprüche

1. Eine monolithische Arzneiform enthaltend
• wenigstens einen ersten Wirkstoff und einen zweiten Wirkstoff, wobei der erste Wirkstoff bei einem pH Wert von 7 und 22°C eine Löslichkeit von weniger als 0,01 mg/ml aufweist, und wobei der zweite Wirkstoff eine Löslichkeit bei pH 7 und 22°C aufweist, die die Löslichkeit des ersten Wirkstoffs bei pH 7 und 22°C um wenigstens eine Zehnerpotenz überschreitet, und
• wenigstens einen Emulgator, wobei der Emulgator durch Reaktion von Mono-, Di-, und/oder Triglyceriden mit Polyethylenglykol hergestellt wird, wobei die Polyethylenglykolkette den hydrophilen Teil des Emulgators darstellt und mit einem lipophilen Teil verbunden ist, und die Polyethylenglykolkette wenigstens 4 Kettenglieder aufweist, wobei der Emulgator ein Strukturmotiv folgender Formel aufweist
R¹-O-[CH₂-CH₂-O]ₙ-R²
wobei R₁ und R₂ unabhängig voneinander Wasserstoff, Alkyl, Glycerid oder Polyalkylenoxid sind und n eine ganze Zahl von wenigstens 4 ist; und
• wenigstens ein Retardierungsmittel,
wobei die Summe der Anteile an Retardierungsmittel und Emulgator an der Arzneiform wenigstens 40 Gew.-% beträgt und wobei das Masseverhältnis von zweitem Wirkstoff zu Retardierungsmittel weniger als 1:1 beträgt.

2. Arzneiform nach Anspruch 1 , wobei die Summe der Anteile an Retardierungsmittel und Emulgator an der Arzneiform wenigstens 48 Gew.-% beträgt.

3. Arzneiform nach Anspruch 1 oder Anspruch 2, wobei der erste Wirkstoff in einer Masse im Verhältnis zur Masse des Emulgators von höchstens 1:2 vorliegt.

4. Arzneiform nach wenigstens einem der vorhergehenden Ansprüche, wobei das Retardierungsmittel ausgewählt ist aus Methylcellulose, Hydroxypropylmethylcellulose und Mischungen daraus.

5. Arzneiform nach wenigstens einem der vorhergehenden Ansprüche, wobei der erste Wirkstoff wenigstens eine Aminogruppe aufweist und bei pH 1 und 22°C eine Löslichkeit von mindestens 0.5 mg/ml hat.

6. Arzneiform nach wenigstens einem der vorhergehenden Ansprüche, wobei der zweite Wirkstoff eine Eliminationshalbwertszeit von mehr als 2 Stunden aufweist.

7. Arzneiform nach wenigstens einem der vorhergehenden Ansprüche, wobei die Arzneiform als Magenverweilform ausgestaltet ist, die für wenigstens 2 Stunden im Magen verweilt, bevor sie in den Darm gelangt.

8. Arzneiform nach wenigstens einem der vorhergehenden Ansprüche, wobei die Arzneiform als Tablette ausgestaltet ist, und wobei die Tablette eine Härte von wenigstens 40 N und höchstens 130 N aufweist.

9. Verfahren zur Herstellung einer Arzneiform nach wenigstens einem der vorhergehenden Ansprüche mit den Schritten:
a. Mischen von Komponenten der Arzneiform;
b. Granulieren der Komponenten; und
c. Herstellung einer monolithischen Arzneiform aus dem Granulat.

10. Arzneiform nach einem der Ansprüche 1 bis 8 zur Verwendung in einem Therapieverfahren.

## Claims

1. A monolithic pharmaceutical form containing
▪ at least one first active ingredient and one second active ingredient, wherein the first active ingredient at a pH value of 7 and 22°C has a solubility of less than 0.01 mg/ml, and wherein the second active ingredient at pH 7 and 22°C has a solubility which exceeds the solubility of the first active ingredient at pH 7 and 22°C by at least one decimal power, and
▪ at least one emulsifier, wherein the emulsifier is prepared by reaction of mono-, di- and/or triglycerides with polyethylene glycol, wherein the polyethylene glycol chain is the hydrophilic part of the emulsifier and is connected with a lipophilic part, and the polyethylene glycol chain comprises at least 4 chain members, wherein the emulsifier has a structural motif of the following formula
R¹-O-[CH₂-CH₂-O]ₙ-R²
wherein R¹ and R² independently of each other are hydrogen, alkyl, glyceride or polyalkylene oxide and n is an integer of at least 4; and
▪ at least one retarding agent,
wherein the sum of the portions of retarding agent and emulsifier of the pharmaceutical form is at least 40 % by weight and wherein the mass ratio of second active ingredient to retarding agent is less than 1:1.

2. The pharmaceutical form according to claim 1, wherein the sum of the portions of retarding agent and emulsifier of the pharmaceutical form is at least 48 % by weight.

3. The pharmaceutical form according to claim 1 or claim 2, wherein the first active ingredient is present in a mass in a ratio to the mass of the emulsifier of at most 1:2.

4. The pharmaceutical form according to at least one of the preceding claims, wherein the retarding agent is selected from methylcellulose, hydroxypropyl methylcellulose and mixtures thereof.

5. The pharmaceutical form according to at least one of the preceding claims, wherein the first active ingredient comprises at least one amino group and at pH 1 and 22°C has a solubility of at least 0.5 mg/ml.

6. The pharmaceutical form according to at least one of the preceding claims, wherein the second active ingredient has a half-life period of elimination of longer than 2 hours.

7. The pharmaceutical form according to at least one of the preceding claims, wherein the pharmaceutical form is designed as a form with a prolonged gastric residence time having a residence time in the stomach of at least 2 hours before entering the intestine.

8. The pharmaceutical form according to at least one of the preceding claims, wherein the pharmaceutical form is designed as a tablet, and wherein the tablet has a hardness of at least 40 N and at most 130 N.

9. A method for the production of a pharmaceutical form according to at least one of the preceding claims with the steps:
a. mixing of components of the pharmaceutical form;
b. granulating of the components; and
c. producing of a monolithic pharmaceutical form from the granules.

10. The pharmaceutical form according to one of claims 1 to 8 for the use in a therapeutic method.

## Revendications

1. Forme galénique monolithique contenant
• au moins une première substance active et une seconde substance active, dans laquelle la première substance active présente à une valeur pH de 7 et à 22 °C une solubilité de moins de 0,01 mg/ml, et dans laquelle la seconde substance active présente une solubilité à une valeur de pH de 7 et à 22 °C qui dépasse la solubilité de la première substance active à une valeur de pH de 7 et à 22 °C d'au moins une puissance de dix, et
• au moins un émulsifiant, dans lequel l'émulsifiant est fabriqué par la réaction de mono-, di- et/ou triglycérides avec du polyéthylène glycol, dans lequel la chaîne de polyéthylène glycol représente la partie hydrophile de l'émulsifiant et est reliée à une partie lipophile, et la chaîne de polyéthylène glycol présente au moins 4 maillons de chaîne, dans lequel l'émulsifiant présente un motif de structure de formule suivante
R¹-O-[CH₂-CH₂-O]ₙ-R²
dans lequel R¹ et R² sont indépendamment l'un de l'autre de l'hydrogène, de l'alkyle, du glycéride ou de l'oxyde de polyalkylène et n est un nombre entier d'au moins 4 ; et
• au moins un moyen de retardement,
dans lequel la somme des parts de moyen de retardement et d'émulsifiant au niveau de la forme galénique s'élève au moins à 40 % en poids et dans lequel le rapport de masse entre la seconde substance active et le moyen de retardement s'élève à moins de 1:1.

2. Forme galénique selon la revendication 1, dans laquelle la somme des parts de moyen de retardement et d'émulsifiant au niveau de la forme galénique s'élève à au moins 48 % en poids.

3. Forme galénique selon la revendication 1 ou la revendication 2, dans laquelle la première substance active dans une masse se présente dans le rapport à la masse de l'émulsifiant au plus à 1:2.

4. Forme galénique selon au moins l'une quelconque des revendications précédentes, dans laquelle le moyen de retardement est sélectionné parmi la méthylcellulose, l'hydroxypropylméthylcellulose et des mélanges de celles-ci.

5. Forme galénique selon au moins l'une quelconque des revendications précédentes, dans laquelle la première substance active présente au moins un groupe amino et a à une valeur de pH de 1 et à 22 °C une solubilité d'au moins 0,5 mg/ml.

6. Forme galénique selon au moins l'une quelconque des revendications précédentes, dans laquelle la seconde substance active présente une demi-vie d'élimination de plus de 2 heures.

7. Forme galénique selon au moins l'une quelconque des revendications précédentes, dans laquelle la forme galénique est configurée comme forme de séjour dans l'estomac qui séjourne pendant au moins 2 heures dans l'estomac avant qu'elle ne parvienne dans l'intestin.

8. Forme galénique selon au moins l'une quelconque des revendications précédentes, dans laquelle la forme galénique est configurée comme comprimé, et dans laquelle la tablette présente une dureté d'au moins 40 N et au plus 130 N.

9. Procédé de fabrication d'une forme galénique selon au moins l'une quelconque des revendications précédentes avec les étapes :
a. le mélange de composants de la forme galénique ;
b. la granulation des composants ; et
c. la fabrication d'une forme galénique monolithique à partir du granulat.

10. Forme galénique selon l'une quelconque des revendications 1 à 8 pour l'utilisation dans un procédé thérapeutique.
